# EUROPEAN PATENT APPLICATION

(11) **EP 4 782 465 A1**
(43) Date of publication of application: **29.07.2026**
(21) Application number: 24867551.4
(22) Date of filing: 20.09.2024
(51) Int. Cl.: C07K 19/00, A61K 38/28, A61K 38/16, A61P 3/10, C12N 15/62

(54) **PHARMACEUTICAL COMPOSITION CONTAINING HUMAN INSULIN ANALOGUE FUSION PROTEIN AND MEDICAL USE THEREOF**

(30) Priority: 20.09.2023 CN 202311219649
(71) Applicant: Shanghai Mabgen Biotech Ltd., Shanghai 201206 (CN)
(72) Inventor: WU, Hui, Shanghai 201206 (CN); ZHAO, Zhilong, Shanghai 201206 (CN); LI, Lei, Shanghai 201206 (CN)
(74) Representative: Regimbeau
(86) International application number: PCT/CN2024/120026
(87) International publication number: WO 2025/061146

(57) **Abstract**

A pharmaceutical composition containing a human insulin analogue fusion protein and the use thereof. The pharmaceutical composition has a good biological activity and stability.

## Description

### TECHNICAL FIELD

The present disclosure relates to the field of pharmaceutical formulations and particularly relates to a pharmaceutical composition comprising a human insulin analog fusion protein and pharmaceutical use thereof.

### BACKGROUND

Diabetes is a chronic metabolic disease in which the metabolism of glucose, proteins, and lipids in the human body is disordered due to insufficient insulin secretion in the body. It is mainly classified into insulin-dependent diabetes (type I diabetes) and non-insulin-dependent diabetes (type II diabetes). Globally, 90-95% of diabetes patients have type II diabetes, which is caused by impaired pancreatic β cell function and long-term insulin resistance, and is primarily characterized by a lack of insulin levels in the body, decreased insulin sensitivity, and high blood glucose concentrations in the plasma. Studies have shown that type II diabetes is associated with a variety of high-risk complications, and can lead to cardiovascular diseases, kidney failure, blindness, amputation, and many other complications.

Approved diabetes drugs on the market mainly include chemically synthesized small-molecule oral hypoglycemic drugs such as biguanides, sulfonyl compounds, insulin sensitizers, and α-glycosides, as well as injectable peptide hypoglycemic drugs such as glucagon-like peptide-1 (GLP-1) analogs. For all type I diabetes cases and some terminal type II diabetes cases, only insulin injection-related drugs can well control the blood glucose levels.

Currently, approved insulin analog products on the market are mainly divided into two categories: rapid-acting insulin and long-acting basal insulin. Rapid-acting insulins that are already available on the market include insulin lispro, insulin aspart, etc. Rapid-acting insulins are primarily administered to patients via subcutaneous injection before meals. They have insulin molecular structures that are engineered to inhibit the formation of natural insulin hexamers, thus showing pharmacokinetic characteristics of rapid onset and quick clearance after administration via subcutaneous injection. Insulin lispro achieves its inhibitory effect on the formation of insulin hexamers through the switching of the amino acids at positions 28 and 29 at the terminus of the natural insulin B-chain, and insulin aspart inhibits the formation of hexamers through the replacement of proline with aspartic acid at position 28 of the insulin B-chain and through charge repulsion interactions. Approved long-acting basal insulins on the market include insulin glargine, insulin detemir, and insulin degludec. Insulin glargine has positively charged arginine that is introduced into the insulin molecular skeleton to raise the molecular isoelectric point to neutrality, so that after administration via subcutaneous injection, the insulin analog forms a precipitate at the site of administration, and the precipitate dissolves in a sustained-release manner, thereby achieving significantly extended *in vivo* pharmacokinetic properties. Insulin detemir and insulin degludec have long-chain fatty acid modifications that are introduced into the lysine side chain at the terminus of the B-chain, and achieve extended *in vivo* pharmacokinetic properties by forming supramolecular polymers at the site of administration and by reversibly binding to human serum albumin in the plasma. Currently, insulin glargine and insulin degludec are administered once daily, while insulin detemir is administered once or twice daily.

However, rapid-acting insulin products may cause adverse reactions such as hypoglycemia and severe hypoglycemia symptoms, and patients need to be injected with insulin drugs once or even multiple times daily, leading to inconvenience, heavy treatment burdens, and decreased quality of life. Moreover, existing long-acting basal insulin products need to be administered via subcutaneous injection once daily, which is also a relatively high injection frequency. Therefore, clinical patients need ultra-long-acting basal insulin products with a lower injection frequency and higher safety, which can significantly improve patient compliance and convenience. WO2023174370 provides such an ultra-long-acting basal insulin product.

However, due to their large molecular weights and complex structures, fusion protein drugs are prone to degradation, aggregation, unwanted chemical modifications, or the like, which make them unstable. It is particularly important to develop stable formulations of fusion protein drugs that make the fusion proteins suitable for administration and keep them stable during storage and subsequent use so that they can produce better effects. For fusion proteins of new insulin analogs, there is still a need to develop suitable pharmaceutical compositions.

### SUMMARY

The present disclosure provides a pharmaceutical composition comprising a human insulin analog fusion protein, e.g., a fusion protein comprising a human insulin analog and an IgG Fc region. The composition has excellent stability.

The present disclosure provides a pharmaceutical composition comprising:
a) a fusion protein of a human insulin analog, and
b) a buffer that is one or more selected from the group consisting of histidine or a salt thereof, acetic acid or a salt thereof, succinic acid or a salt thereof, a citrate, and a phosphate.

In some embodiments, the buffer is one or more selected from the group consisting of histidine or a salt thereof, acetic acid or a salt thereof, and a phosphate. In some embodiments, the buffer is one or more selected from the group consisting of histidine or a salt thereof and a phosphate. In some embodiments, the buffer is one or more selected from the group consisting of histidine, acetic acid, and disodium hydrogen phosphate. In some embodiments, the buffer is one or more selected from the group consisting of histidine, acetic acid-histidine, and disodium hydrogen phosphate. In some embodiments, the buffer is a phosphate. In some embodiments, the buffer is acetic acid-histidine. In some embodiments, the buffer is one or more selected from the group consisting of histidine and disodium hydrogen phosphate. In some embodiments, the buffer is histidine and disodium hydrogen phosphate. In some embodiments, the buffer is histidine.

In some embodiments, the aforementioned fusion protein comprises an insulin analog, wherein: the insulin analog has the following general formula: B₁-L₁-A₁,
wherein B₁ is an insulin B-chain analog comprising the following amino acid sequence:
FVX₁QHLCGX₂HLVEALX₃X₄VCGERGFX₅X₆X₇X₈X₉ (SEQ ID NO: 43), wherein X₁ is selected from the group consisting of N, G, and K; X₂ is selected from the group consisting of S and E; X₃ is selected from the group consisting of Y, H, and E; X₄ is selected from the group consisting of H, R, L, and E; X₅ is selected from the group consisting of F and H; X₆ is selected from the group consisting of G, T, S, H, V, Y, and absent; X₇ is selected from the group consisting of G, E, P, K, D, S, H, and absent; X₈ is selected from the group consisting of G, E, K, P, Q, D, H, and absent; and X₉ is selected from the group consisting of G, T, S, E, K, A, and absent;
A₁ is an insulin A-chain analog comprising the following amino acid sequence:
   GIVEQCCZ₁SICSLZ₂QLENYCZ₃Z₄ (SEQ ID NO: 44), wherein Z₁ is selected from the group consisting of T, H, and E, Z₂ is selected from the group consisting of Y, D, S, and E, and Z₃ is selected from the group consisting of G and N; Z₄ is any natural amino acid or absent, and when Z₃ is N, Z₄ is not G or N.
   L₁ is a linker.

In some embodiments, the aforementioned fusion protein comprises an insulin analog, wherein: the insulin analog has the following general formula: B₁-L₁-A₁,
wherein B₁ is an insulin B-chain analog comprising the following amino acid sequence:
FVX₁QHLCGX₂HLVEALX₃X₄VCGERGFX₅Y (SEQ ID NO: 1), wherein X₁ is selected from the group consisting of N, G, and K, X₂ is selected from the group consisting of S and E, X₃ is selected from the group consisting of Y, H, and E, X₄ is selected from the group consisting of H, R, L, and E, and X₅ is selected from the group consisting of F and H;
A₁ is an insulin A-chain analog comprising the following amino acid sequence:
   GIVEQCCZ₁SICSLZ₂QLENYCZ₃ (SEQ ID NO: 2), wherein Z₁ is selected from the group consisting of T, H, and E, Z₂ is selected from the group consisting of Y, D, S, and E, and Z₃ is selected from the group consisting of G and N;
   L₁ is a linker.

In some embodiments, B₁ comprises (or is) the amino acid sequence set forth in SEQ ID NO: 43 or SEQ ID NO: 1, which comprises at least 1, at least 2, at least 3, at least 4, or 5 amino acid modifications at X₁, X₂, X₃, X₄, and X₅. In some specific embodiments, X₁ is selected from the group consisting of N and K, X₂ is selected from the group consisting of S and E, X₃ is selected from the group consisting of Y and H, X₄ is L, and/or X₅ is H. In some specific embodiments, X₂ is E, and X₅ is H. In some specific embodiments, X₅ is H. In some specific embodiments, X₃ is H, and X₅ is H. In some specific embodiments, X₁ is K, X₂ is E, and X₅ is H.

In some embodiments, A₁ comprises (or is) the amino acid sequence set forth in SEQ ID NO: 44 or SEQ ID NO: 2, which comprises at least 1, at least 2, or 3 amino acid modifications at Z₁, Z₂, and Z₃. In some specific embodiments, Z₁ is selected from the group consisting of T, H, and E, Z₂ is E, and Z₃ is selected from the group consisting of G and N. In some specific embodiments, Z₁ is E, Z₂ is E, and Z₃ is G. In some specific embodiments, Z₁ is H, Z₂ is E, and Z₃ is G. In some specific embodiments, Z₂ is E. In some specific embodiments, Z₁ is E, and Z₂ is E.

In some embodiments, in the insulin analog, B₁ comprises (or is) the sequence of SEQ ID NO: 43 or SEQ ID NO: 1, and A₁ comprises (or is) the amino acid sequence set forth in SEQ ID NO: 44 or SEQ ID NO: 2. In some specific embodiments, X₁ is selected from the group consisting of N and K, X₂ is selected from the group consisting of S and E, X₃ is selected from the group consisting of Y and H, X₄ is L, and X₅ is H; and Z₁ is selected from the group consisting of T, H, and E, Z₂ is E, and Z₃ is selected from the group consisting of G and N. In some specific embodiments, X₂ is E, X₅ is H, Z₁ is E, Z₂ is E, and Z₃ is G. In some specific embodiments, X₅ is H, Z₁ is H, Z₂ is E, and Z₃ is G. In some specific embodiments, X₃ is H, X₅ is H, and Z₂ is E. In some specific embodiments, X₁ is K, X₂ is E, X₅ is H, Z₁ is E, Z₂ is E, and Z₃ is G. In some specific embodiments, X₂ is E, X₅ is H, Z₁ is E, Z₂ is E, and Z₃ is G. In some specific embodiments, X₂ is E, X₅ is H, Z₁ is E, and Z₂ is E.

In some specific embodiments, X₁ is N or K, X₂ is E, X₃ is Y, X₄ is L, X₅ is H, Z₁ is E, Z₂ is E, and Z₃ is G;
X₁ is N, X₂ is E, X₃ is Y, X₄ is L, X₅ is H, Z₁ is E, Z₂ is E, and Z₃ is N;
X₁ is N, X₂ is S, X₃ is Y, X₄ is L, X₅ is H, Z₁ is H, Z₂ is E, and Z₃ is G;
X₁ is N, X₂ is S, X₃ is H, X₄ is L, X₅ is H, Z₁ is T, Z₂ is E, and Z₃ is G; or
X₁ is N, X₂ is E, X₃ is Y, X₄ is L, X₅ is H, Z₁ is E, Z₂ is E, and Z₃ is G.

In the above embodiments, X₆-X₉ or X₇-X₉ may each be G; or X₆-X₉ or X₇-X₉ may be absent. In the above embodiments, Z₄ may be absent; or when Z₃ is N, Z₄ is an amino acid other than G, N, S, V, L, or P.

In some embodiments, in the insulin analog, B₁ comprises (or is) the amino acid sequence of any one of SEQ ID NOs: 31-36:

| | |
|---|---|
| FVNQHLCGEHLVEALYLVCGERGFHY | (SEQ ID NO: 31); |
| FVNQHLCGSHLVEALYLVCGERGFHY | (SEQ ID NO: 32); |
| FVNQHLCGSHLVEALHLVCGERGFHY | (SEQ ID NO: 33); |
| FVKQHLCGEHLVEALYLVCGERGFHY | (SEQ ID NO: 34); |
| FVNQHLCGEHLVEALYLVCGERGFHY | (SEQ ID NO: 35); |
| FVNQHLCGEHLVEALYLVCGERGFHY | (SEQ ID NO: 36). |

In some embodiments, in the insulin analog, A₁ comprises (or is) the amino acid sequence of any one of SEQ ID NOs: 37-42:

| | |
|---|---|
| GIVEQCCESICSLEQLENYCG | (SEQ ID NO: 37); |
| GIVEQCCHSICSLEQLENYCG | (SEQ ID NO: 38); |
| GIVEQCCTSICSLEQLENYCN | (SEQ ID NO: 39); |
| GIVEQCCESICSLEQLENYCG | (SEQ ID NO: 40); |
| GIVEQCCESICSLEQLENYCG | (SEQ ID NO: 41); |
| GIVEQCCESICSLEQLENYCN | (SEQ ID NO: 42). |

In some embodiments, in the insulin analog, B₁ and A₁ comprise (or are) the amino acid sequences set forth in any one of the following groups: SEQ ID NOs: 31 and 37; SEQ ID NOs: 32 and 38; SEQ ID NOs: 33 and 39; SEQ ID NOs: 34 and 40; SEQ ID NOs: 35 and 41; and SEQ ID NOs: 36 and 42.

In some embodiments, the linker represented by L₁ is a polypeptide capable of achieving a linking function, e.g., a flexible polypeptide. In some specific embodiments, the linker represented by L₁ comprises (or is) the following sequence: including but not limited to G₄S (SEQ ID NO: 53), GS, GAP, ASGS (SEQ ID NO: 54), and poly-guanine (poly G). In some specific embodiments, the linker represented by L₁ comprises (or is) (GₘSₚ)ₙ, wherein each m is independently selected from the group consisting of integers from 1 to 10 (e.g., 1, 2, 3, 4, 5, or 6), each n is independently selected from the group consisting of integers from 1 to 10 (e.g., 1, 2, 3, 4, 5, or 6), and each p is independently selected from the group consisting of 0-4. In some specific embodiments, the GS or poly G linker comprises at least 5 Gs.

In the present disclosure, the GS linker encompasses any type of linker that comprises the amino acids G and S.

In some specific embodiments, the linker represented by L₁ comprises (or is) GGSGGGG (SEQ ID NO: 45), GSGGGG (SEQ ID NO: 46), GGGGG (SEQ ID NO: 47), GGGGGGSGGGG (SEQ ID NO: 3), or GGGGGSGGGG (SEQ ID NO: 52).

In some embodiments, any one of the aforementioned fusion proteins further comprises a C₁ domain, and the C₁ domain is capable of extending the half-life of the insulin analog.

In some embodiments, any one of the aforementioned fusion proteins further comprises a C₁ domain, and the C₁ is selected from the group consisting of, for example, an Fc region of an immunoglobulin (e.g., an IgG (e.g., IgG1, IgG2, IgG3, or IgG4 subtype), IgA1, IgA2, IgD, IgE, or IgM), an HSA protein or polypeptide, an HSA-binding domain (e.g., an anti-HSA antibody or an antigen-binding fragment thereof), an XTEN peptide, a glycine-rich homo-amino-acid polymer (HAP), a PAS polypeptide, an elastin-like polypeptide (ELP), a CTP peptide, and a gelatin-like protein (GLK) polymer.

In some specific embodiments, the XTEN peptide is an unstructured, hydrophilic long peptide comprising varying percentages of six amino acids: Ala, Glu, Gly, Ser, and Thr, e.g., the XTEN^{™} peptide (Amunix Operating Inc.). In some specific embodiments, the PAS polypeptide is a hydrophilic, uncharged polypeptide consisting of Pro, Ala, and Ser residues that is at least about 100, 200, 300, 400, 500, or 600 amino acids in length.

In some embodiments, one fusion protein comprises 1 or more (e.g., 2, 3, or 4) insulin analogs and one C₁.

In some specific embodiments, C₁ is selected from the group consisting of Fc regions of human IgG1, IgG2, and IgG4. In some specific embodiments, the Fc region comprises a mutation selected from the group consisting of: L234A/L235A on IgG1; V234A/G237A/P238S/H268A/V309L/A330S/P331S on IgG2; F234A/L235A on IgG4; S228P or S228P/F234A/L235A on IgG4; N297A on IgG1, IgG2, IgG3, or IgG4; V234A/G237A on IgG2; K214T/E233P/L234V/L235A/G236 deletion/A327G/P331A/D365E/L358MV309L/A330S/P331S on IgG1; L234F/L235E/D265A on IgG1; L234A/L235A/G237A/P238S/H268A/A330S/P331S on IgG1; S228P/F234A/L235A/G237A/P238S on IgG4; and S228P/F234A/L235A/G236 deletion/G237A/P238S on IgG4. A hybrid IgG2/4 Fc domain may also be used, e.g., an Fc comprising residues 117-260 from IgG2 and residues 261-447 from IgG4. In some specific embodiments, the Fc region of human IgG4 comprises mutation(s) S228P, F234A, L235A, and/or K447A. In some specific embodiments, the Fc region of human IgG1 comprises mutations L234A/L235A or L234A/L235A/P329G. In some embodiments, the mutation sites of the Fc region described above are numbered according to the EU numbering scheme.

In some specific embodiments, C₁ is selected from the group consisting of an amino acid sequence comprising (or as set forth in) any one of SEQ ID NOs: 17, 18, and 48-51, and a sequence having at least 80%, 90%, 95%, 96%, 97%, 98%, or 99% identity thereto.
> Human IgG1 Fc region 1 
EPKSCDKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPI
EKTISKAKGQPREPQVYTLPPSREEMTKNQVSLTCLVKGFYPSDIAVEWESNGQPENNYK
TTPPVLDSDGSFFLYSKLTVDKSRWQQGNVFSCSVMHEALHNHYTQKSLSLSPGK
(SEQ ID NO: 48);
> Human IgG1 Fc region 2 
EPKSCDKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPI
EKTISKAKGQPREPQVYTLPPSRDELTKNQVSLTCLVKGFYPSDIAVEWESNGQPENNYK
TTPPVLDSDGSFFLYSKLTVDKSRWQQGNVFSCSVMHEALHNHYTQKSLSLSPGK
(SEQ ID NO: 49);
> Human IgG4 Fc region 1 
ESKYGPPCPPCPAPEFEGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGK
(SEQ ID NO: 50);
> Human IgG4 Fc region 2

ESKYGPPCPPCPAPEAAGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGK
(SEQ ID NO: 51).

In some embodiments, any one of the aforementioned fusion proteins further comprises L₂; the linker represented by L₂ is a polypeptide capable of achieving a linking function (e.g., a flexible polypeptide), and C₁ forms the fusion protein with B₁-L₁-A₁ via L₂. In some specific embodiments, the linker represented by L₂ comprises (or is) the following sequence: including but not limited to G₄S, GS, GAP, ASGS, poly-guanine (poly G), (GₘQ)ₙ, (GₘA)ₙ, (GₘQᵢ)ₙ, (GₘAᵢ)ₙ, or (PGPQ)ₛ (SEQ ID NO: 55), wherein each m is independently selected from the group consisting of integers from 1 to 10 (e.g., 1, 2, 3, 4, 5, or 6), each n is independently selected from the group consisting of integers from 1 to 10 (e.g., 1, 2, 3, 4, 5, or 6), each i is independently selected from the group consisting of 0-4 (e.g., 0, 1, 2, 3, or 4), and s is selected from the group consisting of integers from 1 to 10 (e.g., 1, 2, 3, 4, 5, or 6). In some specific embodiments, L₂ comprises (or is) the amino acid sequence set forth in any one of SEQ ID NOs: 10-16.

In some embodiments, in any one of the aforementioned fusion proteins, C₁ is located at the N-terminus of the insulin analog. In some other embodiments, in the fusion protein, C₁ is located at the C-terminus of the insulin analog. In some specific embodiments, the fusion protein is, from the N-terminus to the C-terminus, B₁-L₁-A₁-L₂-C₁, C₁-L₂-B₁-L₁-A₁, A₁-L₁-B₁-L₂-C₁, or C₁-L₂-A₁-L₁-B₁.

In some embodiments, the insulin analog is an insulin receptor agonist having insulin receptor agonistic activity.

In some embodiments, any one of the aforementioned fusion proteins is a monomer, a dimer, or a multimer. In some specific embodiments, the dimer is a homodimer, wherein the amino acid sequences of the two fusion proteins that constitute the dimer are identical or substantially identical. In some other specific embodiments, the dimer is a heterodimer, wherein the amino acid sequences of the two fusion proteins that constitute the dimer are different.

In some embodiments, any one of the aforementioned fusion proteins comprises (or is) the amino acid sequence set forth in any one of SEQ ID NOs: 19-27, or a sequence having at least 80%, 90%, 95%, 96%, 97%, 98%, or 99% identity thereto.

In some embodiments, any one of the aforementioned fusion proteins comprises (or is) the amino acid sequence set forth in SEQ ID NO: 28.

In some embodiments, any one of the aforementioned fusion proteins (e.g., the sequence of any one of SEQ ID NOs: 19-27, e.g., SEQ ID NO: 19, 20, 21, or 27) has a reduced ability to promote cell proliferation, and thus has a reduced risk of inducing tumorigenesis and/or tumor progression. The cell is, for example, a cell expressing IGF-1R on the surface. In some embodiments, the reduction means that the fusion protein, compared to natural human insulin, has a cell (e.g., MCF-7) proliferation promotion EC₅₀ value that is increased by a factor of 1 to 10, 1 to 100, 1 to 1000, 1 to 10000, 100 to 500, or 200 to 400; for example, the EC₅₀ is increased by a factor of about 100, about 200, about 300, about 400, about 500, about 600, about 700, about 800, about 900, or about 1000. In some specific embodiments, the ability of the fusion protein 1 set forth in SEQ ID NO: 19 to promote cell proliferation is about 200 times lower than that of natural human insulin. The cell (e.g., MCF-7) proliferation EC₅₀ can be determined using the method in Example 5 of the present disclosure.

In some embodiments, any one of the aforementioned fusion proteins (e.g., the sequence of any one of SEQ ID NOs: 19-27, e.g., SEQ ID NO: 19 or 20) has a half-life of 1 day to 10 days, 2 days to 9 days, 2 days to 8 days, 3 days to 7 days, e.g., a half-life of about 1 day, about 2 days, about 2.5 days, about 3 days, about 3.5 days, about 4 days, about 4.5 days, about 5 days, about 5.5 days, about 6 days, about 6.5 days, about 7 days, about 7.5 days, about 8 days, about 8.5 days, about 9 days, about 9.5 days, or about 10 days. In some specific embodiments, the fusion protein 1 set forth in SEQ ID NO: 19 has a half-life of about 4.5 days. The half-life of the protein can be determined using the method in Example 6 of the present disclosure.

In some embodiments, the fusion protein in the pharmaceutical composition is at a concentration of 0.01 mg/mL-500 mg/mL, e.g., 0.05 mg/mL-450 mg/mL, 0.05 mg/mL-400 mg/mL, 0.05 mg/mL-350 mg/mL, 0.05 mg/mL-300 mg/mL, 0.05 mg/mL-250 mg/mL, 0.05 mg/mL-200 mg/mL, 0.05 mg/mL-150 mg/mL, 0.05 mg/mL-140 mg/mL, 0.05 mg/mL-130 mg/mL, 0.05 mg/mL-120 mg/mL, 0.05 mg/mL-110 mg/mL, 0.05 mg/mL-100 mg/mL, 0.1 mg/mL-400 mg/mL, 0.1 mg/mL-350 mg/mL, 0.1 mg/mL-300 mg/mL, 0.1 mg/mL-250 mg/mL, 0.1 mg/mL-200 mg/mL, 0.1 mg/mL-150 mg/mL, 0.1 mg/mL-140 mg/mL, 0.1 mg/mL-130 mg/mL, 0.1 mg/mL-120 mg/mL, 0.1 mg/mL-110 mg/mL, 0.1 mg/mL-100 mg/mL, 0.5 mg/mL-350 mg/mL, 0.5 mg/mL-300 mg/mL, 0.5 mg/mL-250 mg/mL, 0.5 mg/mL-200 mg/mL, 0.5 mg/mL-150 mg/mL, 0.5 mg/mL-140 mg/mL, 0.5 mg/mL-130 mg/mL, 0.5 mg/mL-120 mg/mL, 0.5 mg/mL-110 mg/mL, 0.5 mg/mL-100 mg/mL, 1 mg/mL-300 mg/mL, 1 mg/mL-250 mg/mL, 1 mg/mL-200 mg/mL, 1 mg/mL-150 mg/mL, 1 mg/mL-140 mg/mL, 1 mg/mL-130 mg/mL, 1 mg/mL-120 mg/mL, 1 mg/mL-110 mg/mL, 1 mg/mL-100 mg/mL, 1 mg/mL-95 mg/mL, 1 mg/mL-90 mg/mL, 1 mg/mL-85 mg/mL, 1 mg/mL-80 mg/mL, 1 mg/mL-75 mg/mL, 1 mg/mL-70 mg/mL, 1 mg/mL-65 mg/mL, 1 mg/mL-60 mg/mL, 1 mg/mL-55 mg/mL, 1 mg/mL-50 mg/mL, 1 mg/mL-45 mg/mL, 1 mg/mL-40 mg/mL, 1 mg/mL-35 mg/mL, 1 mg/mL-30 mg/mL, 1 mg/mL-25 mg/mL, 1 mg/mL-20 mg/mL, 1 mg/mL-15 mg/mL, 5 mg/mL-90 mg/mL, 5 mg/mL-85 mg/mL, 5 mg/mL-80 mg/mL, 5 mg/mL-75 mg/mL, 5 mg/mL-70 mg/mL, 5 mg/mL-65 mg/mL, 5 mg/mL-60 mg/mL, 5 mg/mL-55 mg/mL, 5 mg/mL-50 mg/mL, 5 mg/mL-45 mg/mL, 5 mg/mL-40 mg/mL, 5 mg/mL-35 mg/mL, 5 mg/mL-30 mg/mL, 5 mg/mL-25 mg/mL, 5 mg/mL-20 mg/mL, 5 mg/mL-15 mg/mL, 8 mg/mL-12 mg/mL, 30 mg/mL-250 mg/mL, 30 mg/mL-200 mg/mL, 30 mg/mL-190 mg/mL, 30 mg/mL-180 mg/mL, 30 mg/mL-170 mg/mL, 30 mg/mL-160 mg/mL, 30 mg/mL-150 mg/mL, 30 mg/mL-140 mg/mL, 30 mg/mL-130 mg/mL, 30 mg/mL-120 mg/mL, 30 mg/mL-110 mg/mL, 30 mg/mL-100 mg/mL, 50 mg/mL-200 mg/mL, 50 mg/mL-190 mg/mL, 50 mg/mL-180 mg/mL, 50 mg/mL-170 mg/mL, 50 mg/mL-160 mg/mL, 50 mg/mL-150 mg/mL, 50 mg/mL-140 mg/mL, 50 mg/mL-130 mg/mL, 50 mg/mL-120 mg/mL, 50 mg/mL-110 mg/mL, 50 mg/mL-100 mg/mL, 70 mg/mL-180 mg/mL, 70 mg/mL-170 mg/mL, 70 mg/mL-160 mg/mL, 70 mg/mL-150 mg/mL, 70 mg/mL-140 mg/mL, 70 mg/mL-130 mg/mL, 70 mg/mL-120 mg/mL, 70 mg/mL-110 mg/mL, 70 mg/mL-100 mg/mL, 90 mg/mL-110 mg/mL, or 95 mg/mL-105 mg/mL. In some embodiments, the fusion protein in the pharmaceutical composition is at a concentration of 0.1 mg/mL-400 mg/mL. In some embodiments, the fusion protein in the pharmaceutical composition is at a concentration of 0.5 mg/mL-200 mg/mL. In some embodiments, the fusion protein in the pharmaceutical composition is at a concentration of 1 mg/mL-150 mg/mL. In some embodiments, the fusion protein in the pharmaceutical composition is at a concentration of 5 mg/mL-45 mg/mL. In some embodiments, the fusion protein in the pharmaceutical composition is at a concentration of 50 mg/mL-110 mg/mL. In some embodiments, the fusion protein in the pharmaceutical composition is at a concentration of about 1 mg/mL, about 5 mg/mL, about 10 mg/mL, about 15 mg/mL, about 20 mg/mL, about 25 mg/mL, about 30 mg/mL, about 40 mg/mL, about 45 mg/mL, about 50 mg/mL, about 55 mg/mL, about 60 mg/mL, about 70 mg/mL, about 75 mg/mL, about 80 mg/mL, about 85 mg/mL, about 90 mg/mL, about 95 mg/mL, about 100 mg/mL, about 105 mg/mL, about 110 mg/mL, about 115 mg/mL, about 120 mg/mL, about 125 mg/mL, about 130 mg/mL, about 135 mg/mL, about 140 mg/mL, about 145 mg/mL, or about 150 mg/mL. In some embodiments, the fusion protein in the pharmaceutical composition is at a concentration of 1 mg/mL, about 20 mg/mL, about 40 mg/mL, about 50 mg/mL, about 60 mg/mL, about 80 mg/mL, about 100 mg/mL, about 120 mg/mL, or 150 mg/mL. In some embodiments, the fusion protein in the pharmaceutical composition is at a concentration of about 50 mg/mL, about 60 mg/mL, about 70 mg/mL, about 80 mg/mL, about 90 mg/mL, about 100 mg/mL, about 110 mg/mL, or about 120 mg/mL. In some embodiments, the fusion protein in the pharmaceutical composition is at a concentration of about 5 mg/mL, about 10 mg/mL, about 20 mg/mL, about 30 mg/mL, about 40 mg/mL, about 50 mg/mL, or about 60 mg/mL. In some embodiments, the fusion protein in the pharmaceutical composition is at a concentration of about 40 mg/mL, about 60 mg/mL, or about 100 mg/mL.

In some embodiments, the buffer in the pharmaceutical composition is at a concentration of 1 mM-200 mM, e.g., 5 mM-190 mM, 5 mM-180 mM, 5 mM-170 mM, 5 mM-160 mM, 5 mM-150 mM, 5 mM-140 mM, 5 mM-130 mM, 5 mM-120 mM, 5 mM-110 mM, 5 mM-100 mM, 5 mM-90 mM, 5 mM-80 mM, 10 mM-180 mM, 10 mM-170 mM, 10 mM-160 mM, 10 mM-150 mM, 10 mM-140 mM, 10 mM-130 mM, 10 mM-120 mM, 10 mM-110 mM, 10 mM-100 mM, 10 mM-90 mM, 10 mM-80 mM, 20 mM-170 mM, 20 mM-160 mM, 20 mM-150 mM, 20 mM-140 mM, 20 mM-130 mM, 20 mM-120 mM, 20 mM-110 mM, 20 mM-100 mM, 20 mM-90 mM, 20 mM-80 mM, 40 mM-160 mM, 40 mM-150 mM, 40 mM-140 mM, 40 mM-130 mM, 40 mM-120 mM, 40 mM-110 mM, 40 mM-100 mM, 40 mM-90 mM, 40 mM-80 mM, 60 mM-150 mM, 60 mM-140 mM, 60 mM-130 mM, 60 mM-120 mM, 60 mM-110 mM, 60 mM-100 mM, 60 mM-90 mM, or 60 mM-80 mM. In some embodiments, the buffer in the pharmaceutical composition is at a concentration of 10 mM-150 mM or 5 mM-150 mM. In some embodiments, the buffer in the pharmaceutical composition is at a concentration of 40 mM-120 mM or 10 mM-120 mM. In some embodiments, the buffer in the pharmaceutical composition is at a concentration of 60 mM-100 mM or 10 mM-100 mM. In some embodiments, the buffer in the pharmaceutical composition is at a concentration of about 10 mM, about 20 mM, about 30 mM, about 40 mM, about 50 mM, about 60 mM, about 65 mM, about 70 mM, about 75 mM, about 80 mM, about 85 mM, about 90 mM, about 95 mM, or about 100 mM. In some embodiments, the buffer in the pharmaceutical composition is at a concentration of about 10 mM, about 40 mM, or about 80 mM.

In some embodiments, the pharmaceutical composition further comprises a surfactant. In some embodiments, the surfactant is one or more selected from the group consisting of polysorbate and poloxamer. In some embodiments, the polysorbate is one or more selected from the group consisting of polysorbate 20 and polysorbate 80. In some embodiments, the surfactant is polysorbate 80. In some embodiments, the surfactant is poloxamer, e.g., poloxamer 188.

In some embodiments, the surfactant in the pharmaceutical composition is at a concentration of 0.01 mg/mL-5 mg/mL, e.g., 0.01 mg/mL-5 mg/mL, 0.01 mg/mL-4 mg/mL, 0.01 mg/mL-3 mg/mL, 0.01 mg/mL-2 mg/mL, 0.01 mg/mL-1 mg/mL, 0.01 mg/mL-0.5 mg/mL, 0.05 mg/mL-4 mg/mL, 0.05 mg/mL-3 mg/mL, 0.05 mg/mL-2 mg/mL, 0.05 mg/mL-1 mg/mL, 0.05 mg/mL-0.5 mg/mL, 0.08 mg/mL-3 mg/mL, 0.08 mg/mL-2.5 mg/mL, 0.08 mg/mL-2 mg/mL, 0.08 mg/mL-1.5 mg/mL, 0.08 mg/mL-1 mg/mL, 0.08 mg/mL-0.5 mg/mL, 0.1 mg/mL-2.5 mg/mL, 0.1 mg/mL-2 mg/mL, 0.1 mg/mL-1.5 mg/mL, 0.1 mg/mL-1 mg/mL, 0.1 mg/mL-0.5 mg/mL, 0.2 mg/mL-2.5 mg/mL, 0.2 mg/mL-2 mg/mL, 0.2 mg/mL-1.5 mg/mL, 0.2 mg/mL-1 mg/mL, 0.2 mg/mL-0.5 mg/mL, 0.3 mg/mL-2.5 mg/mL, 0.3 mg/mL-2 mg/mL, 0.3 mg/mL-1.5 mg/mL, 0.3 mg/mL-1 mg/mL, 0.3 mg/mL-0.5 mg/mL, 0.4 mg/mL-2.5 mg/mL, 0.4 mg/mL-2 mg/mL, 0.4 mg/mL-1.5 mg/mL, or 0.4 mg/mL-1 mg/mL. In some embodiments, the surfactant in the pharmaceutical composition is at a concentration of 0.05 mg/mL-3 mg/mL. In some embodiments, the surfactant in the pharmaceutical composition is at a concentration of 0.08 mg/mL-1.5 mg/mL. In some embodiments, the surfactant in the pharmaceutical composition is at a concentration of 0.1 mg/mL-1 mg/mL. In some embodiments, the surfactant in the pharmaceutical composition is at a concentration of about 0.1 mg/mL, about 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL, or about 1 mg/mL. In some embodiments, the surfactant in the pharmaceutical composition is at a concentration of about 0.2 mg/mL or about 0.4 mg/mL.

In some embodiments, the pharmaceutical composition further comprises one or more of a saccharide and a polyol. In some embodiments, the polyol is one or more selected from the group consisting of mannitol, sorbitol, and glycerol. In some embodiments, the saccharide is one or more selected from the group consisting of sucrose and trehalose. In some embodiments, the saccharide is sucrose. In some embodiments, the polyol is sorbitol or glycerol. In some embodiments, the polyol is trehalose. In some embodiments, the polyol is mannitol.

In some embodiments, the saccharide or polyol in the pharmaceutical composition is at a concentration of 1 mg/mL-200 mg/mL, e.g., 1 mg/mL-180 mg/mL, 1 mg/mL-170 mg/mL, 1 mg/mL-160 mg/mL, 1 mg/mL-150 mg/mL, 1 mg/mL-140 mg/mL, 1 mg/mL-130 mg/mL, 1 mg/mL-120 mg/mL, 1 mg/mL-110 mg/mL, 1 mg/mL-100 mg/mL, 1 mg/mL-90 mg/mL, 1 mg/mL-80 mg/mL, 1 mg/mL-70 mg/mL, 1 mg/mL-60 mg/mL, 1 mg/mL-50 mg/mL, 5 mg/mL-160 mg/mL, 5 mg/mL-150 mg/mL, 5 mg/mL-140 mg/mL, 5 mg/mL-130 mg/mL, 5 mg/mL-120 mg/mL, 5 mg/mL-110 mg/mL, 5 mg/mL-100 mg/mL, 5 mg/mL-90 mg/mL, 5 mg/mL-80 mg/mL, 5 mg/mL-70 mg/mL, 5 mg/mL-60 mg/mL, 5 mg/mL-50 mg/mL, 10 mg/mL-150 mg/mL, 10 mg/mL-140 mg/mL, 10 mg/mL-130 mg/mL, 10 mg/mL-120 mg/mL, 10 mg/mL-110 mg/mL, 10 mg/mL-100 mg/mL, 10 mg/mL-90 mg/mL, 10 mg/mL-80 mg/mL, 10 mg/mL-70 mg/mL, 10 mg/mL-60 mg/mL, 10 mg/mL-50 mg/mL, 20 mg/mL-140 mg/mL, 20 mg/mL-130 mg/mL, 20 mg/mL-120 mg/mL, 20 mg/mL-110 mg/mL, 20 mg/mL-100 mg/mL, 20 mg/mL-90 mg/mL, 20 mg/mL-80 mg/mL, 20 mg/mL-70 mg/mL, 20 mg/mL-60 mg/mL, 20 mg/mL-50 mg/mL, 30 mg/mL-130 mg/mL, 30 mg/mL-120 mg/mL, 30 mg/mL-110 mg/mL, 30 mg/mL-100 mg/mL, 30 mg/mL-90 mg/mL, 30 mg/mL-80 mg/mL, 30 mg/mL-70 mg/mL, 30 mg/mL-60 mg/mL, 30 mg/mL-50 mg/mL, 40 mg/mL-120 mg/mL, 40 mg/mL-110 mg/mL, 40 mg/mL-100 mg/mL, 40 mg/mL-90 mg/mL, 40 mg/mL-80 mg/mL, 40 mg/mL-70 mg/mL, 40 mg/mL-60 mg/mL, 40 mg/mL-50 mg/mL, 50 mg/mL-110 mg/mL, 50 mg/mL-100 mg/mL, 50 mg/mL-90 mg/mL, 50 mg/mL-80 mg/mL, 50 mg/mL-75 mg/mL, 60 mg/mL-100 mg/mL, 60 mg/mL-90 mg/mL, 60 mg/mL-80 mg/mL, 60 mg/mL-75 mg/mL, 65 mg/mL-90 mg/mL, 65 mg/mL-80 mg/mL, or 65 mg/mL-75 mg/mL. In some embodiments, the saccharide or polyol in the pharmaceutical composition is at a concentration of 10 mg/mL-150 mg/mL. In some embodiments, the saccharide or polyol in the pharmaceutical composition is at a concentration of 30 mg/mL-120 mg/mL. In some embodiments, the saccharide or polyol in the pharmaceutical composition is at a concentration of 40 mg/mL-100 mg/mL.

In some embodiments, sucrose or trehalose in the pharmaceutical composition is at a concentration of 1 mg/mL-150 mg/mL, e.g., 5 mg/mL-140 mg/mL, 5 mg/mL-130 mg/mL, 5 mg/mL-120 mg/mL, 5 mg/mL-110 mg/mL, 5 mg/mL-100 mg/mL, 5 mg/mL-90 mg/mL, 5 mg/mL-80 mg/mL, 5 mg/mL-70 mg/mL, 10 mg/mL-130 mg/mL, 10 mg/mL-120 mg/mL, 10 mg/mL-110 mg/mL, 10 mg/mL-100 mg/mL, 10 mg/mL-90 mg/mL, 10 mg/mL-80 mg/mL, 10 mg/mL-70 mg/mL, 20 mg/mL-130 mg/mL, 20 mg/mL-120 mg/mL, 20 mg/mL-110 mg/mL, 20 mg/mL-100 mg/mL, 20 mg/mL-90 mg/mL, 20 mg/mL-80 mg/mL, 20 mg/mL-70 mg/mL, 30 mg/mL-130 mg/mL, 30 mg/mL-110 mg/mL, 30 mg/mL-100 mg/mL, 30 mg/mL-90 mg/mL, 30 mg/mL-80 mg/mL, 30 mg/mL-70 mg/mL, 40 mg/mL-120 mg/mL, 40 mg/mL-110 mg/mL, 40 mg/mL-100 mg/mL, 40 mg/mL-90 mg/mL, 40 mg/mL-80 mg/mL, 40 mg/mL-70 mg/mL, 50 mg/mL-120 mg/mL, 50 mg/mL-110 mg/mL, 50 mg/mL-100 mg/mL, 50 mg/mL-100 mg/mL, 50 mg/mL-90 mg/mL, 50 mg/mL-80 mg/mL, 50 mg/mL-70 mg/mL, 60 mg/mL-120 mg/mL, 60 mg/mL-110 mg/mL, 60 mg/mL-100 mg/mL, 60 mg/mL-90 mg/mL, 60 mg/mL-80 mg/mL, 60 mg/mL-70 mg/mL, 65 mg/mL-110 mg/mL, 65 mg/mL-100 mg/mL, 65 mg/mL-90 mg/mL, 65 mg/mL-80 mg/mL, 65 mg/mL-70 mg/mL, 70 mg/mL-110 mg/mL, 70 mg/mL-100 mg/mL, 70 mg/mL-95 mg/mL, 70 mg/mL-90 mg/mL, 75 mg/mL-110 mg/mL, 75 mg/mL-100 mg/mL, 75 mg/mL-95 mg/mL, 75 mg/mL-90 mg/mL, 80 mg/mL-110 mg/mL, 80 mg/mL-100 mg/mL, 80 mg/mL-95 mg/mL, or 80 mg/mL-90 mg/mL. In some embodiments, sucrose in the pharmaceutical composition is at a concentration of 10 mg/mL-120 mg/mL. In some embodiments, sucrose in the pharmaceutical composition is at a concentration of 20 mg/mL-100 mg/mL. In some embodiments, sucrose in the pharmaceutical composition is at a concentration of 50 mg/mL-90 mg/mL. In some embodiments, sucrose in the pharmaceutical composition is at a concentration of about 50 mg/mL, about 55 mg/mL, about 60 mg/mL, about 65 mg/mL, about 70 mg/mL, about 75 mg/mL, about 80 mg/mL, about 85 mg/mL, or about 90 mg/mL. In some embodiments, sucrose in the pharmaceutical composition is at a concentration of about 60 mg/mL, about 70 mg/mL, or about 80 mg/mL.

In some embodiments, trehalose in the pharmaceutical composition is at a concentration of 5 mg/mL-140 mg/mL. In some embodiments, trehalose in the pharmaceutical composition is at a concentration of 30 mg/mL-130 mg/mL. In some embodiments, trehalose in the pharmaceutical composition is at a concentration of 60 mg/mL-120 mg/mL. In some embodiments, trehalose in the pharmaceutical composition is at a concentration of 75 mg/mL-100 mg/mL. In some embodiments, trehalose in the pharmaceutical composition is at a concentration of about 75 mg/mL, about 76 mg/mL, about 77 mg/mL, about 78 mg/mL, about 79 mg/mL, about 80 mg/mL, about 81 mg/mL, about 82 mg/mL, about 83 mg/mL, about 84 mg/mL, about 85 mg/mL, about 86 mg/mL, about 87 mg/mL, about 88 mg/mL, about 89 mg/mL, about 90 mg/mL, about 91 mg/mL, about 92 mg/mL, about 93 mg/mL, about 94 mg/mL, about 95 mg/mL, about 96 mg/mL, about 97 mg/mL, about 98 mg/mL, about 99 mg/mL, or about 100 mg/mL. In some embodiments, trehalose in the pharmaceutical composition is at a concentration of about 88 mg/mL.

In some embodiments, sorbitol or mannitol in the pharmaceutical composition is at a concentration of 1 mg/mL-100 mg/mL, e.g., 5 mg/mL-100 mg/mL, 5 mg/mL-90 mg/mL, 5 mg/mL-80 mg/mL, 5 mg/mL-70 mg/mL, 5 mg/mL-60 mg/mL, 5 mg/mL-50 mg/mL, 10 mg/mL-90 mg/mL, 10 mg/mL-80 mg/mL, 10 mg/mL-70 mg/mL, 10 mg/mL-60 mg/mL, 10 mg/mL-50 mg/mL, 20 mg/mL-70 mg/mL, 20 mg/mL-65 mg/mL, 20 mg/mL-60 mg/mL, 20 mg/mL-55 mg/mL, 20 mg/mL-50 mg/mL, 30 mg/mL-70 mg/mL, 30 mg/mL-65 mg/mL, 30 mg/mL-60 mg/mL, 30 mg/mL-55 mg/mL, 30 mg/mL-50 mg/mL, 35 mg/mL-70 mg/mL, 35 mg/mL-65 mg/mL, 35 mg/mL-60 mg/mL, 35 mg/mL-55 mg/mL, 35 mg/mL-50 mg/mL, 40 mg/mL-60 mg/mL, 40 mg/mL-55 mg/mL, or 40 mg/mL-50 mg/mL.

In some embodiments, sorbitol in the pharmaceutical composition is at a concentration of 10 mg/mL-80 mg/mL. In some embodiments, sorbitol in the pharmaceutical composition is at a concentration of 20 mg/mL-70 mg/mL. In some embodiments, sorbitol in the pharmaceutical composition is at a concentration of 30 mg/mL-60 mg/mL. In some embodiments, sorbitol in the pharmaceutical composition is at a concentration of about 30 mg/mL, about 35 mg/mL, about 40 mg/mL, about 45 mg/mL, about 50 mg/mL, about 55 mg/mL, or about 60 mg/mL. In some embodiments, sorbitol in the pharmaceutical composition is at a concentration of about 45 mg/mL.

In some embodiments, mannitol in the pharmaceutical composition is at a concentration of 10 mg/mL-80 mg/mL. In some embodiments, mannitol in the pharmaceutical composition is at a concentration of 20 mg/mL-70 mg/mL. In some embodiments, mannitol in the pharmaceutical composition is at a concentration of 30 mg/mL-60 mg/mL. In some embodiments, mannitol in the pharmaceutical composition is at a concentration of about 40 mg/mL, about 41 mg/mL, about 42 mg/mL, about 43 mg/mL, about 44 mg/mL, about 45 mg/mL, about 46 mg/mL, about 47 mg/mL, about 48 mg/mL, about 49 mg/mL, about 50 mg/mL, about 51 mg/mL, about 52 mg/mL, about 53 mg/mL, about 54 mg/mL, about 55 mg/mL, about 56 mg/mL, about 57 mg/mL, about 58 mg/mL, about 59 mg/mL, or about 60 mg/mL. In some embodiments, mannitol in the pharmaceutical composition is at a concentration of about 50 mg/mL.

In some embodiments, the pharmaceutical composition further comprises a bacteriostatic agent that comprises or is one or more selected from the group consisting of benzyl alcohol, phenol, and m-cresol. In some embodiments, the bacteriostatic agent comprises or is benzyl alcohol. In some embodiments, the bacteriostatic agent comprises or is phenol. In some embodiments, the bacteriostatic agent comprises or is m-cresol.

In some embodiments, the bacteriostatic agent in the pharmaceutical composition is at a concentration of 0.01 mg/mL-50 mg/mL, e.g., 0.05 mg/mL-45 mg/mL, 0.05 mg/mL-40 mg/mL, 0.05 mg/mL-35 mg/mL, 0.05 mg/mL-30 mg/mL, 0.05 mg/mL-25 mg/mL, 0.05 mg/mL-20 mg/mL, 0.05 mg/mL-15 mg/mL, 0.05 mg/mL-10 mg/mL, 0.05 mg/mL-5 mg/mL, 0.1 mg/mL-40 mg/mL, 0.1 mg/mL-35 mg/mL, 0.1 mg/mL-30 mg/mL, 0.1 mg/mL-25 mg/mL, 0.1 mg/mL-20 mg/mL, 0.1 mg/mL-15 mg/mL, 0.1 mg/mL-10 mg/mL, 0.1 mg/mL-5 mg/mL, 0.5 mg/mL-35 mg/mL, 0.5 mg/mL-30 mg/mL, 0.5 mg/mL-25 mg/mL, 0.5 mg/mL-20 mg/mL, 0.5 mg/mL-15 mg/mL, 0.5 mg/mL-10 mg/mL, 0.5 mg/mL-5 mg/mL, 1 mg/mL-30 mg/mL, 1 mg/mL-25 mg/mL, 1 mg/mL-20 mg/mL, 1 mg/mL-15 mg/mL, 1 mg/mL-10 mg/mL, 1 mg/mL-5 mg/mL, 2 mg/mL-25 mg/mL, 2 mg/mL-20 mg/mL, 2 mg/mL-15 mg/mL, 2 mg/mL-10 mg/mL, 2 mg/mL-5 mg/mL, 5 mg/mL-20 mg/mL, 5 mg/mL-15 mg/mL, or 5 mg/mL-10 mg/mL. In some embodiments, the bacteriostatic agent in the pharmaceutical composition is at a concentration of 0.05 mg/mL-40 mg/mL. In some embodiments, the bacteriostatic agent in the pharmaceutical composition is at a concentration of 0.1 mg/mL-30 mg/mL. In some embodiments, the bacteriostatic agent in the pharmaceutical composition is at a concentration of 0.5 mg/mL-20 mg/mL.

In some embodiments, benzyl alcohol in the pharmaceutical composition is at a concentration of 0.05 mg/mL-40 mg/mL, e.g., 0.05 mg/mL-35 mg/mL, 0.05 mg/mL-30 mg/mL, 0.05 mg/mL-25 mg/mL, 0.05 mg/mL-20 mg/mL, 0.05 mg/mL-15 mg/mL, 0.05 mg/mL-10 mg/mL, 0.05 mg/mL-5 mg/mL, 0.1 mg/mL-40 mg/mL, 0.1 mg/mL-35 mg/mL, 0.1 mg/mL-30 mg/mL, 0.1 mg/mL-25 mg/mL, 0.1 mg/mL-20 mg/mL, 0.1 mg/mL-15 mg/mL, 0.1 mg/mL-10 mg/mL, 0.5 mg/mL-35 mg/mL, 0.5 mg/mL-30 mg/mL, 0.5 mg/mL-25 mg/mL, 0.5 mg/mL-20 mg/mL, 0.5 mg/mL-15 mg/mL, 0.5 mg/mL-10 mg/mL, 1 mg/mL-30 mg/mL, 1 mg/mL-25 mg/mL, 1 mg/mL-20 mg/mL, 1 mg/mL-15 mg/mL, 1 mg/mL-10 mg/mL, 2 mg/mL-25 mg/mL, 2 mg/mL-20 mg/mL, 2 mg/mL-15 mg/mL, 2 mg/mL-10 mg/mL, 2 mg/mL-5 mg/mL, 5 mg/mL-20 mg/mL, 5 mg/mL-15 mg/mL, or 5 mg/mL-10 mg/mL. In some embodiments, benzyl alcohol in the pharmaceutical composition is at a concentration of 0.1 mg/mL-30 mg/mL. In some embodiments, benzyl alcohol in the pharmaceutical composition is at a concentration of 0.5 mg/mL-20 mg/mL. In some embodiments, benzyl alcohol in the pharmaceutical composition is at a concentration of 1 mg/mL-15 mg/mL. In some embodiments, benzyl alcohol in the pharmaceutical composition is at a concentration of about 1 mg/mL, about 2 mg/mL, about 3 mg/mL, about 4 mg/mL, about 5 mg/mL, about 6 mg/mL, about 7 mg/mL, about 8 mg/mL, about 9 mg/mL, about 10 mg/mL, about 11 mg/mL, about 12 mg/mL, about 13 mg/mL, about 14 mg/mL, or about 15 mg/mL. In some embodiments, benzyl alcohol in the pharmaceutical composition is at a concentration of about 5 mg/mL or about 10 mg/mL.

In some embodiments, phenol in the pharmaceutical composition is at a concentration of 0.05 mg/mL-40 mg/mL, e.g., 0.05 mg/mL-35 mg/mL, 0.05 mg/mL-30 mg/mL, 0.05 mg/mL-25 mg/mL, 0.05 mg/mL-20 mg/mL, 0.05 mg/mL-15 mg/mL, 0.05 mg/mL-10 mg/mL, 0.05 mg/mL-5 mg/mL, 0.1 mg/mL-30 mg/mL, 0.1 mg/mL-25 mg/mL, 0.1 mg/mL-20 mg/mL, 0.1 mg/mL-15 mg/mL, 0.1 mg/mL-10 mg/mL, 0.1 mg/mL-5 mg/mL, 0.5 mg/mL-25 mg/mL, 0.5 mg/mL-20 mg/mL, 0.5 mg/mL-15 mg/mL, 0.5 mg/mL-10 mg/mL, 0.5 mg/mL-5 mg/mL, 1 mg/mL-20 mg/mL, 1 mg/mL-15 mg/mL, 1 mg/mL-10 mg/mL, 1 mg/mL-5 mg/mL, 2 mg/mL-15 mg/mL, 2 mg/mL-10 mg/mL, or 2 mg/mL-5 mg/mL. In some embodiments, phenol in the pharmaceutical composition is at a concentration of 0.1 mg/mL-30 mg/mL. In some embodiments, phenol in the pharmaceutical composition is at a concentration of 0.5 mg/mL-15 mg/mL. In some embodiments, phenol in the pharmaceutical composition is at a concentration of 1 mg/mL-10 mg/mL. In some embodiments, phenol in the pharmaceutical composition is at a concentration of about 1 mg/mL, 1.5 mg/mL, 2 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, or 10 mg/mL. In some embodiments, phenol in the pharmaceutical composition is at a concentration of about 2.5 mg/mL or 5 mg/mL.

In some embodiments, m-cresol in the pharmaceutical composition is at a concentration of 0.01 mg/mL-30 mg/mL, e.g., 0.01 mg/mL-30 mg/mL, 0.01 mg/mL-25 mg/mL, 0.01 mg/mL-20 mg/mL, 0.01 mg/mL-15 mg/mL, 0.01 mg/mL-10 mg/mL, 0.01 mg/mL-5 mg/mL, 0.05 mg/mL-25 mg/mL, 0.05 mg/mL-20 mg/mL, 0.05 mg/mL-15 mg/mL, 0.05 mg/mL-10 mg/mL, 0.05 mg/mL-5 mg/mL, 0.1 mg/mL-20 mg/mL, 0.1 mg/mL-15 mg/mL, 0.1 mg/mL-10 mg/mL, 0.1 mg/mL-5 mg/mL, 0.5 mg/mL-15 mg/mL, 0.5 mg/mL-12 mg/mL, 0.5 mg/mL-10 mg/mL, 0.5 mg/mL-8 mg/mL, 0.5 mg/mL-5 mg/mL, 1 mg/mL-10 mg/mL, 1 mg/mL-8 mg/mL, or 1 mg/mL-5 mg/mL. In some embodiments, m-cresol in the pharmaceutical composition is at a concentration of 0.05 mg/mL-15 mg/mL. In some embodiments, m-cresol in the pharmaceutical composition is at a concentration of 0.1 mg/mL-10 mg/mL. In some embodiments, m-cresol in the pharmaceutical composition is at a concentration of 0.5 mg/mL-5 mg/mL. In some embodiments, m-cresol in the pharmaceutical composition is at a concentration of about 0.5 mg/mL, 1 mg/mL, 1.5 mg/mL, 2 mg/mL, 2.5 mg/mL, 3 mg/mL, 3.5 mg/mL, 4 mg/mL, 4.5 mg/mL, or 5 mg/mL. In some embodiments, m-cresol in the pharmaceutical composition is at a concentration of about 1 mg/mL or 3 mg/mL. In some embodiments, when the bacteriostatic agent in the pharmaceutical composition is benzyl alcohol (e.g., 1 mg/mL-15 mg/mL, e.g., 5 mg/mL-10 mg/mL), the composition further comprises a surfactant (e.g., 0.1 mg/mL-1 mg/mL, e.g., 0.2 mg/mL-0.4 mg/mL), and the surfactant is polysorbate (e.g., polysorbate 80). In some embodiments, when the bacteriostatic agent in the pharmaceutical composition is phenol (e.g., 1 mg/mL-10 mg/mL, e.g., 2.5 mg/mL-5 mg/mL, e.g., about 2.5 mg/mL or about 5 mg/mL), the composition further comprises a surfactant (e.g., 0.4 mg/mL-2.5 mg/mL, e.g., 0.4 mg/mL-1 mg/mL, e.g., about 0.4 mg/mL), and the surfactant is polysorbate, e.g., polysorbate 80.

In some embodiments, the buffer in the pharmaceutical composition has a pH of 5-9.5, e.g., 5.5-9, 5.5-8.5, 5.5-8, 5.5-7.5, 5.5-7, 6-9, 6-8.5, 6-8, 6-7.5, 6-7, 6.5-8.5, 6.5-8, 6.5-7.5, 6.5-7, 7-8, or 7-7.5. In some embodiments, the buffer in the pharmaceutical composition has a pH of 6-9. In some embodiments, the buffer in the pharmaceutical composition has a pH of 6.5-8.5. In some embodiments, the buffer in the pharmaceutical composition has a pH of 7-8. In some embodiments, the buffer in the pharmaceutical composition has a pH of 6.5-7.5. In some embodiments, the buffer in the pharmaceutical composition has a pH of about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, or about 8.0. In some embodiments, the buffer in the pharmaceutical composition has a pH of about 6.5, about 6.6, about 6.7, about 6.8, about 6.9, about 7.0, about 7.1, about 7.2, about 7.3, about 7.4, or about 7.5. In some embodiments, the buffer in the pharmaceutical composition has a pH of about 6.5, about 7.0, or about 7.5.

In some embodiments, the pharmaceutical composition further comprises a pH adjuster, e.g., sodium hydroxide and/or hydrochloric acid.

In some embodiments, there is a difference of no more than ±0.5 between the pH of the pharmaceutical composition and the pH of the buffer that the pharmaceutical composition comprises. In some embodiments, the pharmaceutical composition has a pH of 5-9.5, e.g., 5.5-9, 5.5-8.5, 5.5-8, 5.5-7.5, 5.5-7, 6-9, 6-8.5, 6-8, 6-7.5, 6-7, 6.5-8.5, 6.5-8, 6.5-7.5, 6.5-7, 7-8, or 7-7.5. In some embodiments, the pharmaceutical composition has a pH of 6-9. In some embodiments, the pharmaceutical composition has a pH of 6.5-8.5. In some embodiments, the pharmaceutical composition has a pH of 7-8. In some embodiments, the buffer in the pharmaceutical composition has a pH of 6.5-7.5. In some embodiments, the pharmaceutical composition has a pH of about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, or about 8.0. In some embodiments, the pharmaceutical composition has a pH of about 6.5, about 6.6, about 6.7, about 6.8, about 6.9, about 7.0, about 7.1, about 7.2, about 7.3, about 7.4, or about 7.5. In some embodiments, the pharmaceutical composition has a pH of about 6.5, about 7.0, or about 7.5.

In some embodiments, the present disclosure provides a pharmaceutical composition comprising a fusion protein of a human insulin analog (e.g., shown by the amino acid sequence of SEQ ID NO: 19), comprising any one of the following groups 1)-8):
1) the fusion protein, and
   a buffer that is one or more selected from the group consisting of histidine or a salt thereof and a phosphate, e.g., one or more selected from the group consisting of histidine, acetic acid-histidine, and disodium hydrogen phosphate, e.g., histidine;
2) the fusion protein,
   a buffer that is one or more selected from the group consisting of histidine or a salt thereof and a phosphate, e.g., one or more selected from the group consisting of histidine, acetic acid-histidine, and disodium hydrogen phosphate, e.g., histidine,
   polysorbate or poloxamer, and
   sucrose, sorbitol, or glycerol;
   optionally, the composition further comprising a bacteriostatic agent that comprises or is, for example, one or more selected from the group consisting of benzyl alcohol, phenol, and m-cresol;
3) the fusion protein,
   a buffer that is histidine,
   polysorbate, and
   mannitol or trehalose;
   optionally, the composition further comprising a bacteriostatic agent that comprises or is, for example, one or more selected from the group consisting of benzyl alcohol, phenol, and m-cresol;
4) the fusion protein, and
   a buffer that is one or more selected from the group consisting of histidine or a salt thereof and a phosphate, e.g., one or more selected from the group consisting of histidine, acetic acid-histidine, and disodium hydrogen phosphate, e.g., histidine;
   the pharmaceutical composition having a pH of 7-8, e.g., about 7.0, about 7.1, about 7.2, about 7.3, about 7.4, about 7.5, about 7.6, about 7.7, about 7.8, about 7.9, or about 8.0;
5) the fusion protein, and
   a buffer that is one or more selected from the group consisting of histidine or a salt thereof and a phosphate, e.g., one or more selected from the group consisting of histidine, acetic acid-histidine, and disodium hydrogen phosphate, e.g., histidine;
   the pharmaceutical composition having a pH of 6.5-7.5, e.g., about 6.5, about 6.6, about 6.7, about 6.8, about 6.9, about 7.0, about 7.1, about 7.2, about 7.3, about 7.4, or about 7.5;
6) the fusion protein,
   a buffer that is, e.g., one or more selected from the group consisting of histidine or a salt thereof and a phosphate, e.g., one or more selected from the group consisting of histidine, acetic acid-histidine, and disodium hydrogen phosphate, e.g., histidine, and
   a bacteriostatic agent, wherein i) when the bacteriostatic agent comprises or is benzyl alcohol (e.g., 1 mg/mL-15 mg/mL, e.g., 5 mg/mL-10 mg/mL), the composition further comprises a surfactant (e.g., 0.1 mg/mL-1 mg/mL, e.g., 0.2 mg/mL-0.4 mg/mL), and the surfactant is polysorbate (e.g., polysorbate 80) or poloxamer (e.g., poloxamer 188); or
   ii) when the bacteriostatic agent comprises or is phenol (e.g., 1 mg/mL-10 mg/mL, e.g., 2.5 mg/mL-5 mg/mL, e.g., about 2.5 mg/mL or about 5 mg/mL), the composition further comprises a surfactant (e.g., 0.4 mg/mL-2.5 mg/mL, e.g., 0.4 mg/mL-1 mg/mL, e.g., about 0.4 mg/mL), and the surfactant is polysorbate or poloxamer, e.g., polysorbate 80 or poloxamer;
7) the fusion protein,
   a buffer that is one or more selected from the group consisting of histidine, acetic acid-histidine, and disodium hydrogen phosphate, e.g., histidine, and
   a bacteriostatic agent, wherein i) when the bacteriostatic agent comprises or is benzyl alcohol (e.g., 1 mg/mL-15 mg/mL, e.g., 5 mg/mL-10 mg/mL), the composition further comprises a surfactant (e.g., 0.1 mg/mL-1 mg/mL, e.g., 0.2 mg/mL-0.4 mg/mL), and the surfactant is polysorbate (e.g., polysorbate 80) or poloxamer (e.g., poloxamer 188); or
   ii) when the bacteriostatic agent comprises or is phenol (e.g., 1 mg/mL-10 mg/mL, e.g., 2.5 mg/mL-5 mg/mL, e.g., about 2.5 mg/mL or about 5 mg/mL), the composition further comprises a surfactant (e.g., 0.4 mg/mL-2.5 mg/mL, e.g., 0.4 mg/mL-1 mg/mL, e.g., about 0.4 mg/mL), and the surfactant is polysorbate or poloxamer, e.g., polysorbate 80;
   the pharmaceutical composition having a pH of 7-8, e.g., about 7.0, about 7.1, about 7.2, about 7.3, about 7.4, about 7.5, about 7.6, about 7.7, about 7.8, about 7.9, or about 8.0; and
8) the fusion protein,
   a buffer that is one or more selected from the group consisting of histidine, acetic acid-histidine, and disodium hydrogen phosphate, e.g., histidine, and
   a bacteriostatic agent, wherein i) when the bacteriostatic agent comprises or is benzyl alcohol (e.g., 1 mg/mL-15 mg/mL, e.g., 5 mg/mL-10 mg/mL), the composition further comprises a surfactant (e.g., 0.1 mg/mL-1 mg/mL, e.g., 0.2 mg/mL-0.4 mg/mL), and the surfactant is polysorbate (e.g., polysorbate 80) or poloxamer (e.g., poloxamer 188); or
   ii) when the bacteriostatic agent comprises or is phenol (e.g., 1 mg/mL-10 mg/mL, e.g., 2.5 mg/mL-5 mg/mL, e.g., about 2.5 mg/mL or about 5 mg/mL), the composition further comprises a surfactant (e.g., 0.4 mg/mL-2.5 mg/mL, e.g., 0.4 mg/mL-1 mg/mL, e.g., about 0.4 mg/mL), and the surfactant is polysorbate or poloxamer, e.g., polysorbate 80;
   the pharmaceutical composition having a pH of 6.5-7.5, e.g., about 6.5, about 6.6, about 6.7, about 6.8, about 6.9, about 7.0, about 7.1, about 7.2, about 7.3, about 7.4, or about 7.5.

In some embodiments, the present disclosure provides a pharmaceutical composition comprising a fusion protein of a human insulin analog (e.g., shown by the amino acid sequence of SEQ ID NO: 19), comprising or being any one of the following groups 1)-22):
1) the fusion protein, histidine, polysorbate, sucrose, and water for injection;
2) the fusion protein, histidine, polysorbate, sucrose, benzyl alcohol, and water for injection;
3) the fusion protein, histidine, polysorbate, sucrose, phenol, and water for injection;
4) the fusion protein, histidine, polysorbate, sucrose, m-cresol, and water for injection;
5) the fusion protein, histidine, polysorbate, sorbitol, and water for injection;
6) the fusion protein, histidine, polysorbate, sorbitol, benzyl alcohol, and water for injection;
7) the fusion protein, histidine, polysorbate, sorbitol, phenol, and water for injection;
8) the fusion protein, histidine, polysorbate, sorbitol, m-cresol, and water for injection;
9) the fusion protein, histidine, polysorbate, mannitol, and water for injection;
10) the fusion protein, histidine, polysorbate, mannitol, benzyl alcohol, and water for injection;
11) the fusion protein, histidine, polysorbate, mannitol, phenol, and water for injection;
12) the fusion protein, histidine, polysorbate, mannitol, m-cresol, and water for injection;
13) the fusion protein, histidine, polysorbate, trehalose, and water for injection;
14) the fusion protein, histidine, polysorbate, trehalose, benzyl alcohol, and water for injection;
15) the fusion protein, histidine, polysorbate, trehalose, phenol, and water for injection;
16) the fusion protein, histidine, polysorbate, trehalose, m-cresol, and water for injection;
17) the fusion protein, histidine, poloxamer, sucrose, and water for injection;
18) the fusion protein, histidine, poloxamer, sucrose, benzyl alcohol, and water for injection;
19) the fusion protein, histidine, poloxamer, sucrose, phenol, and water for injection;
20) the fusion protein, histidine, poloxamer, sucrose, m-cresol, and water for injection;
21) the pharmaceutical composition in any one of 1)-16), wherein the polysorbate is polysorbate 80; and
22) the pharmaceutical composition in any one of 17)-21), wherein polysorbate is replaced with poloxamer 188.

In some embodiments, the present disclosure provides a pharmaceutical composition comprising a fusion protein of a human insulin analog (e.g., shown by the amino acid sequence of SEQ ID NO: 19), comprising any one of the following groups 1)-12):
1) 0.01 mg/mL-500 mg/mL fusion protein,
   1 mM-200 mM buffer that is one or more selected from the group consisting of histidine or a salt thereof and a phosphate, e.g., one or more selected from the group consisting of histidine and disodium hydrogen phosphate,
   0.01 mg/mL-5 mg/mL polysorbate or poloxamer, and
   1 mg/mL-200 mg/mL sucrose or sorbitol;
   optionally, the pharmaceutical composition further comprising 0.01 mg/mL-50 mg/mL bacteriostatic agent that comprises or is one or more selected from the group consisting of benzyl alcohol, phenol, and m-cresol;
   and the pharmaceutical composition having a pH of 5-9.5;
2) 0.1 mg/mL-400 mg/mL fusion protein,
   5 mM-150 mM buffer that is one or more selected from the group consisting of histidine or a salt thereof and a phosphate, e.g., one or more selected from the group consisting of histidine and disodium hydrogen phosphate,
   0.05 mg/mL-3 mg/mL polysorbate or poloxamer, and
   10 mg/mL-150 mg/mL sucrose or sorbitol;
   optionally, the pharmaceutical composition further comprising 0.05 mg/mL-40 mg/mL bacteriostatic agent that comprises or is one or more selected from the group consisting of benzyl alcohol, phenol, and m-cresol;
   and the pharmaceutical composition having a pH of 6-9;
3) 0.5 mg/mL-200 mg/mL fusion protein,
   10 mM-120 mM buffer that is one or more selected from the group consisting of histidine or a salt thereof and a phosphate, e.g., one or more selected from the group consisting of histidine and disodium hydrogen phosphate,
   0.08 mg/mL-1.5 mg/mL polysorbate or poloxamer, and
   30 mg/mL-120 mg/mL sucrose or sorbitol;
   optionally, the pharmaceutical composition further comprising 0.1 mg/mL-30 mg/mL bacteriostatic agent that comprises or is one or more selected from the group consisting of benzyl alcohol, phenol, and m-cresol;
   and the pharmaceutical composition having a pH of 6.5-8.5;
4) 1 mg/mL-150 mg/mL fusion protein,
   10 mM-100 mM buffer that is one or more selected from the group consisting of histidine or a salt thereof and a phosphate, e.g., one or more selected from the group consisting of histidine and disodium hydrogen phosphate,
   0.1 mg/mL-1 mg/mL polysorbate or poloxamer, and
   40 mg/mL-100 mg/mL sucrose or sorbitol;
   optionally, the pharmaceutical composition further comprising 0.5 mg/mL-20 mg/mL bacteriostatic agent that comprises or is one or more selected from the group consisting of benzyl alcohol, phenol, and m-cresol;
   and the pharmaceutical composition having a pH of 6.5-7.5;
5) 0.01 mg/mL-500 mg/mL fusion protein,
   1 mM-200 mM buffer that is one or more selected from the group consisting of histidine and disodium hydrogen phosphate, e.g., histidine,
   0.01 mg/mL-5 mg/mL polysorbate, and
   1 mg/mL-200 mg/mL sucrose or sorbitol;
   optionally, the pharmaceutical composition further comprising 0.01 mg/mL-50 mg/mL bacteriostatic agent that comprises or is one or more selected from the group consisting of benzyl alcohol, phenol, and m-cresol;
   and the pharmaceutical composition having a pH of 5-9.5;
6) 0.1 mg/mL-400 mg/mL fusion protein,
   10 mM-150 mM buffer that is one or more selected from the group consisting of histidine and disodium hydrogen phosphate, e.g., histidine,
   0.05 mg/mL-3 mg/mL polysorbate, and
   10 mg/mL-150 mg/mL sucrose or sorbitol;
   optionally, the pharmaceutical composition further comprising 0.05 mg/mL-40 mg/mL bacteriostatic agent that comprises or is one or more selected from the group consisting of benzyl alcohol, phenol, and m-cresol;
   and the pharmaceutical composition having a pH of 6-9;
7) 0.5 mg/mL-200 mg/mL fusion protein,
   40 mM-120 mM buffer that is one or more selected from the group consisting of histidine and disodium hydrogen phosphate, e.g., histidine,
   0.08 mg/mL-1.5 mg/mL polysorbate, and
   30 mg/mL-120 mg/mL sucrose or sorbitol;
   optionally, the pharmaceutical composition further comprising 0.1 mg/mL-30 mg/mL bacteriostatic agent that comprises or is one or more selected from the group consisting of benzyl alcohol, phenol, and m-cresol;
   and the pharmaceutical composition having a pH of 6.5-8.5;
8) 1 mg/mL-150 mg/mL fusion protein,
   60 mM-100 mM buffer that is one or more selected from the group consisting of histidine and disodium hydrogen phosphate, e.g., histidine,
   0.1 mg/mL-1 mg/mL polysorbate, and
   40 mg/mL-100 mg/mL sucrose or sorbitol;
   optionally, the pharmaceutical composition further comprising 0.5 mg/mL-20 mg/mL bacteriostatic agent that comprises or is one or more selected from the group consisting of benzyl alcohol, phenol, and m-cresol;
   and the pharmaceutical composition having a pH of 7-8;
9) the pharmaceutical composition in 1)-8), wherein sucrose or sorbitol is replaced with mannitol;
10) the pharmaceutical composition in 1)-8), wherein sucrose or sorbitol is replaced with trehalose;
11) the pharmaceutical composition in 1)-4), wherein the buffer is histidine; and
12) the pharmaceutical composition in 1)-11), further comprising water for injection.

In some embodiments, the present disclosure provides a pharmaceutical composition comprising any one of the aforementioned human insulin analog fusion proteins (e.g., shown by the amino acid sequence of SEQ ID NO: 19), comprising or being any one of the following groups 1)-37):
1) 1-200 mg/mL fusion protein,
   about 80 mM or about 40 mM histidine,
   0.1 mg/mL-1 mg/mL polysorbate 80 or poloxamer 188, and
   about 70 mg/mL sucrose, or about 60 mg/mL or about 45 mg/mL sorbitol;
   the pharmaceutical composition having a pH of 7-8 or 6.5-7.5; and
   optionally, the pharmaceutical composition further comprising 0.5 mg/mL-20 mg/mL bacteriostatic agent that comprises or is one or more selected from the group consisting of benzyl alcohol, phenol, and m-cresol;
2) 1-200 mg/mL fusion protein,
   about 80 mM histidine,
   0.1 mg/mL-1 mg/mL polysorbate 80, and
   about 88 mg/mL trehalose or about 50 mg/mL mannitol;
   the pharmaceutical composition having a pH of 7-8 or 6.5-7.5; and
   optionally, the pharmaceutical composition further comprising 0.5 mg/mL-20 mg/mL bacteriostatic agent that comprises or is one or more selected from the group consisting of benzyl alcohol, phenol, and m-cresol;
3) 1-200 mg/mL fusion protein, e.g., about 1 mg/mL, about 20 mg/mL, about 40 mg/mL, about 50 mg/mL, about 60 mg/mL, about 80 mg/mL, about 100 mg/mL, about 120 mg/mL, or 150 mg/mL,
   about 80 mM histidine,
   about 0.2 mg/mL or about 0.4 mg/mL polysorbate 80, and
   about 70 mg/mL sucrose or about 45 mg/mL sorbitol;
   the pharmaceutical composition having a pH of about 6.5, about 7.0, or about 7.5; and
   optionally, the pharmaceutical composition further comprising a bacteriostatic agent comprising about 5 mg/mL or about 10 mg/mL benzyl alcohol, about 2.5 mg/mL or about 5 mg/mL phenol, or about 1 mg/mL or about 3 mg/mL m-cresol;
4) 1-200 mg/mL fusion protein, e.g., about 1 mg/mL, about 20 mg/mL, about 40 mg/mL, about 50 mg/mL, about 60 mg/mL, about 80 mg/mL, about 100 mg/mL, about 120 mg/mL, or 150 mg/mL,
   about 80 mM histidine,
   about 0.2 mg/mL or about 0.4 mg/mL polysorbate 80, and
   about 88 mg/mL trehalose or about 50 mg/mL mannitol;
   the pharmaceutical composition having a pH of about 6.5, about 7.0, or about 7.5; and
   optionally, the pharmaceutical composition further comprising a bacteriostatic agent selected from the group consisting of about 5 mg/mL or about 10 mg/mL benzyl alcohol, about 2.5 mg/mL or about 5 mg/mL phenol, and about 1 mg/mL or about 3 mg/mL m-cresol;
5) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.2 mg/mL polysorbate 80, and
   about 70 mg/mL sucrose;
   the pharmaceutical composition having a pH of about 7.0; and
   optionally, the pharmaceutical composition further comprising about 5 mg/mL or about 10 mg/mL benzyl alcohol;
6) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.4 mg/mL polysorbate 80, and
   about 70 mg/mL sucrose;
   the pharmaceutical composition having a pH of about 7.0; and
   optionally, the pharmaceutical composition further comprising about 5 mg/mL or about 10 mg/mL benzyl alcohol;
7) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.2 mg/mL polysorbate 80, and
   about 70 mg/mL sucrose;
   the pharmaceutical composition having a pH of about 7.5; and
   optionally, the pharmaceutical composition further comprising about 5 mg/mL or about 10 mg/mL benzyl alcohol;
8) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.4 mg/mL polysorbate 80, and
   about 70 mg/mL sucrose;
   the pharmaceutical composition having a pH of about 7.5; and
   optionally, the pharmaceutical composition further comprising about 5 mg/mL or about 10 mg/mL benzyl alcohol;
9) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.2 mg/mL polysorbate 80, and
   about 70 mg/mL sucrose;
   the pharmaceutical composition having a pH of about 7.0; and
   optionally, the pharmaceutical composition further comprising about 2.5 mg/mL or about 5 mg/mL phenol;
10) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.4 mg/mL polysorbate 80, and
   about 70 mg/mL sucrose;
   the pharmaceutical composition having a pH of about 7.0; and
   optionally, the pharmaceutical composition further comprising about 2.5 mg/mL or about 5 mg/mL phenol;
11) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.2 mg/mL polysorbate 80, and
   about 70 mg/mL sucrose;
   the pharmaceutical composition having a pH of about 7.5; and
   optionally, the pharmaceutical composition further comprising about 2.5 mg/mL or about 5 mg/mL phenol;
12) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.4 mg/mL polysorbate 80, and
   about 70 mg/mL sucrose;
   the pharmaceutical composition having a pH of about 7.5; and
   optionally, the pharmaceutical composition further comprising about 2.5 mg/mL or about 5 mg/mL phenol;
13) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.2 mg/mL polysorbate 80, and
   about 70 mg/mL sucrose;
   the pharmaceutical composition having a pH of about 7.0; and
   optionally, the pharmaceutical composition further comprising about 1 mg/mL or about 3 mg/mL m-cresol;
14) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.4 mg/mL polysorbate 80, and
   about 70 mg/mL sucrose;
   the pharmaceutical composition having a pH of about 7.0; and
   optionally, the pharmaceutical composition further comprising about 1 mg/mL or about 3 mg/mL m-cresol;
15) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.2 mg/mL polysorbate 80, and
   about 70 mg/mL sucrose;
   the pharmaceutical composition having a pH of about 7.5; and
   optionally, the pharmaceutical composition further comprising about 1 mg/mL or about 3 mg/mL m-cresol;
16) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.4 mg/mL polysorbate 80, and
   about 70 mg/mL sucrose;
   the pharmaceutical composition having a pH of about 7.5; and
   optionally, the pharmaceutical composition further comprising about 1 mg/mL or about 3 mg/mL m-cresol;
17) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.2 mg/mL polysorbate 80, and
   about 45 mg/mL sorbitol;
   the pharmaceutical composition having a pH of about 7.0; and
   optionally, the pharmaceutical composition further comprising about 5 mg/mL or about 10 mg/mL benzyl alcohol;
18) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.4 mg/mL polysorbate 80, and
   about 45 mg/mL sorbitol;
   the pharmaceutical composition having a pH of about 7.0; and
   optionally, the pharmaceutical composition further comprising about 5 mg/mL or about 10 mg/mL benzyl alcohol;
19) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.2 mg/mL polysorbate 80, and
   about 45 mg/mL sorbitol;
   the pharmaceutical composition having a pH of about 7.5; and
   optionally, the pharmaceutical composition further comprising about 5 mg/mL or about 10 mg/mL benzyl alcohol;
20) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.4 mg/mL polysorbate 80, and
   about 45 mg/mL sorbitol;
   the pharmaceutical composition having a pH of about 7.5; and
   optionally, the pharmaceutical composition further comprising about 5 mg/mL or about 10 mg/mL benzyl alcohol;
21) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.2 mg/mL polysorbate 80, and
   about 45 mg/mL sorbitol;
   the pharmaceutical composition having a pH of about 7.0; and
   optionally, the pharmaceutical composition further comprising about 2.5 mg/mL or about 5 mg/mL phenol;
22) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.4 mg/mL polysorbate 80, and
   about 45 mg/mL sorbitol;
   the pharmaceutical composition having a pH of about 7.0; and
   optionally, the pharmaceutical composition further comprising about 2.5 mg/mL or about 5 mg/mL phenol;
23) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.2 mg/mL polysorbate 80, and
   about 45 mg/mL sorbitol;
   the pharmaceutical composition having a pH of about 7.5; and
   optionally, the pharmaceutical composition further comprising about 2.5 mg/mL or about 5 mg/mL phenol;
24) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.4 mg/mL polysorbate 80, and
   about 45 mg/mL sorbitol;
   the pharmaceutical composition having a pH of about 7.5; and
   optionally, the pharmaceutical composition further comprising about 2.5 mg/mL or about 5 mg/mL phenol;
25) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.2 mg/mL polysorbate 80, and
   about 45 mg/mL sorbitol;
   the pharmaceutical composition having a pH of about 7.0; and
   optionally, the pharmaceutical composition further comprising about 1 mg/mL or about 3 mg/mL m-cresol;
26) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.4 mg/mL polysorbate 80, and
   about 45 mg/mL sorbitol;
   the pharmaceutical composition having a pH of about 7.0; and
   optionally, the pharmaceutical composition further comprising about 1 mg/mL or about 3 mg/mL m-cresol;
27) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.2 mg/mL polysorbate 80, and
   about 45 mg/mL sorbitol;
   the pharmaceutical composition having a pH of about 7.5; and
   optionally, the pharmaceutical composition further comprising about 1 mg/mL or about 3 mg/mL m-cresol;
28) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 80 mM histidine,
   about 0.4 mg/mL polysorbate 80, and
   about 45 mg/mL sorbitol;
   the pharmaceutical composition having a pH of about 7.5; and
   optionally, the pharmaceutical composition further comprising about 1 mg/mL or about 3 mg/mL m-cresol;
29) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 40 mM histidine,
   about 0.4 mg/mL poloxamer 188, and
   about 60 mg/mL sucrose;
   the pharmaceutical composition having a pH of about 6.0, about 7.0, or about 7.5; and
   optionally, the pharmaceutical composition further comprising one or more of benzyl alcohol, phenol, and m-cresol, e.g., about 5 mg/mL or about 10 mg/mL benzyl alcohol, about 2.5 mg/mL or about 5 mg/mL phenol, or about 1 mg/mL or about 3 mg/mL m-cresol;
30) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 40 mM histidine,
   about 0.4 mg/mL poloxamer 188, and
   about 60 mg/mL sucrose;
   the pharmaceutical composition having a pH of about 7.0; and
   optionally, the pharmaceutical composition further comprising one or more of benzyl alcohol, phenol, and m-cresol, e.g., about 5 mg/mL or about 10 mg/mL benzyl alcohol, about 2.5 mg/mL or about 5 mg/mL phenol, or about 1 mg/mL or about 3 mg/mL m-cresol;
31) about 1 mg/mL, about 20 mg/mL, about 40 mg/mL, about 50 mg/mL, about 60 mg/mL, about 80 mg/mL, about 100 mg/mL, about 120 mg/mL, or 150 mg/mL fusion protein, e.g., about 40 mg/mL, about 60 mg/mL, or about 100 mg/mL,
   about 40 mM histidine,
   about 0.4 mg/mL poloxamer 188, and
   about 60 mg/mL sucrose;
   the pharmaceutical composition having a pH of about 7.5; and
   optionally, the pharmaceutical composition further comprising one or more of benzyl alcohol, phenol, and m-cresol, e.g., about 5 mg/mL or about 10 mg/mL benzyl alcohol, about 2.5 mg/mL or about 5 mg/mL phenol, or about 1 mg/mL or about 3 mg/mL m-cresol;
32) the pharmaceutical composition in any one of groups 3)-16), wherein polysorbate 80 is replaced with poloxamer 188;
33) the pharmaceutical composition in any one of groups 5)-16), wherein sucrose is replaced with 80 mg/mL or 60 mg/mL sucrose;
34) the pharmaceutical composition in any one of groups 1)-16), wherein sucrose is replaced with 88 mg/mL trehalose;
35) the pharmaceutical composition in any one of groups 1)-16), wherein sucrose is replaced with 50 mg/mL mannitol;
36) the pharmaceutical composition in any one of groups 1)-35), wherein the pharmaceutical composition may also have a pH of about 6.5; and
37) the pharmaceutical composition in any one of groups 1)-36), with a final volume of 1 mL, the composition being brought to volume (1 mL) with water for injection if necessary.

The pharmaceutical composition of the present disclosure already has sufficient stability for the manufacture of a drug and can remain stable during long-term storage.

In some embodiments, the pharmaceutical composition remains stable at 2-8 °C for at least 3 months, at least 6 months, at least 12 months, at least 18 months, at least 24 months, or at least 36 months. In some embodiments, the pharmaceutical composition remains stable at 25 °C for at least 3 months, at least 6 months, at least 12 months, at least 18 months, or at least 24 months. In some embodiments, the pharmaceutical composition remains stable at 40 °C for at least 7 days, at least 14 days, at least 28 days, at least 1 month, at least 3 months, at least 6 months, at least 12 months, at least 18 months, or at least 24 months.

The present disclosure provides a method for preparing the aforementioned pharmaceutical composition, comprising a step of dissolving the aforementioned fusion protein.

The pharmaceutical composition of the present disclosure may be further made into a freeze-dried formulation to facilitate drug transportation.

The present disclosure further provides a freeze-dried formulation, wherein the freeze-dried formulation is capable of forming the pharmaceutical composition according to any one of the above after reconstitution.

The present disclosure further provides a freeze-dried formulation, wherein the freeze-dried formulation is obtained by lyophilizing the pharmaceutical composition according to any one of the above.

The present disclosure provides a reconstituted solution, wherein the reconstituted solution is prepared by reconstituting the aforementioned freeze-dried formulation. In certain embodiments, the reconstituted solution is selected from the group consisting of, but is not limited to, water for injection, normal saline, and a glucose solution.

The present disclosure further provides a product comprising a container, wherein the container contains the aforementioned pharmaceutical composition, the aforementioned freeze-dried formulation, or the aforementioned reconstituted solution. In certain embodiments, the container is a tubular injection vial made of neutral borosilicate glass. In certain embodiments, the product comprises a package insert.

The present disclosure further provides the pharmaceutical composition or the freeze-dried formulation or the reconstituted solution of the freeze-dried formulation as a medicament for use in treating or alleviating a disease or disorder.

### Method for Treating Disease and Pharmaceutical Use

The present disclosure provides pharmaceutical use of the aforementioned pharmaceutical composition or freeze-dried formulation or reconstituted solution of the freeze-dried formulation in the manufacture of a medicament for preventing, treating, or alleviating the disease or symptom described above.

The present disclosure provides use and a method of the aforementioned pharmaceutical composition or freeze-dried formulation or reconstituted solution of the freeze-dried formulation in the prevention, treatment, or alleviation of a disease or symptom.

The present disclosure provides a method for preventing, treating, or alleviating a disease or symptom, comprising administering to a patient or subject a prophylactically and/or therapeutically effective amount of the aforementioned pharmaceutical composition or freeze-dried formulation or reconstituted solution of the freeze-dried formulation.

In some embodiments, a method for administering an effective amount of the fusion protein or the insulin analog of the present disclosure to a subject in need thereof for preventing, treating, or alleviating a disease or symptom is included.

In some embodiments, the disease or symptom is selected from the group consisting of non-insulin-dependent diabetes, insulin-dependent diabetes, and other metabolic diseases. The subject is not treated or is treated with oral drugs (such as sulfonylureas, metformin, thiazolidinediones such as pioglitazone, and α-glucosidase inhibitors such as acarbose) and/or non-insulin injectable agents (including incretin-based therapies such as DPP-4 inhibitors and GLP-1R agonists).

In some embodiments, the disease or symptom is selected from the group consisting of diabetes, obesity, dyslipidemia, and/or metabolic syndrome.

In some embodiments, the disease or symptom is a complication of diabetes, or diabetes-related heart disease, stroke, nephropathy, retinopathy, neuropathy, or kidney disease.

In some embodiments, the disease or symptom is selected from the group consisting of hyperglycemia, type II diabetes, impaired glucose tolerance, type I diabetes, obesity, syndrome X, and dyslipidemia.

In some embodiments, a method for administering an effective amount of the fusion protein or the insulin analog of the present disclosure to a subject in need thereof for lowering blood glucose levels is provided.

The pharmaceutical composition of the present disclosure may be used for treating a patient in need of such treatment by parenteral administration. For parenteral routes of administration, subcutaneous injection, intramuscular injection, or intravenous injection may be selected.

### Terminology

In order to facilitate the understanding of the present disclosure, certain technical and scientific terms are specifically defined below. Unless otherwise specifically defined in the present disclosure, all other technical and scientific terms used in the present disclosure have the meanings generally understood by those of ordinary skill in the art to which the present disclosure pertains. The three-letter and single-letter codes for amino acids used in the present disclosure are as described in J. Biol. Chem, 243, p3558 (1968).

"Insulin" includes naturally occurring insulin (e.g., human insulin). Human insulin consists of two polypeptide chains, referred to as an A-chain and a B-chain, which contain 21 and 30 amino acid residues, respectively, and are linked by two cysteine disulfide bonds. An exemplary A-chain is set forth in SEQ ID NO: 29 of the present disclosure, and an exemplary B-chain is set forth in SEQ ID NO: 30 of the present disclosure.

"Insulin analog" includes polypeptides that, through the removal and/or substitution (replacement) of one or more amino acid residues present in natural insulin and/or through the addition of one or more amino acid residues, have a molecular structure that can formally be derived from naturally occurring insulin (e.g., the human insulin structure). The added and/or substituted amino acid residues may be codable amino acid residues or other naturally occurring amino acid residues or purely synthetic amino acid residues.

"Insulin A-chain analog" refers to an analog that, compared to the A-chain of human insulin, comprises one or more amino acid substitutions, deletions, and/or extensions (additions) on the A-chain.

"Insulin B-chain analog" refers to an analog that, compared to the B-chain of human insulin, comprises one or more amino acid substitutions, deletions, and/or extensions (additions) on the B-chain.

"Glucose-responsive insulin (GRI)" can regulate the release and action of insulin based on blood glucose levels, thereby achieving a reduction in the incidence of hypoglycemia and making blood glucose control more stable.

The term "insulin receptor agonist" refers to a protein that binds to and activates an insulin receptor, resulting in a decrease in blood glucose levels and/or inhibition of hepatic glucose output; it is characterized in that it can be tested and measured using known techniques (such as those shown in the studies described in the examples of the present disclosure). In some embodiments, the insulin receptor agonist is formed by directly or indirectly linking the insulin A-chain analog and the insulin B-chain analog (e.g., by a linker).

"Sequence" is generally understood to include both related amino acid sequences and nucleic acid or nucleotide sequences encoding the sequences, unless a further limited interpretation is required in the present disclosure.

"Homology" or "identity" refers to the sequence similarity between two polynucleotide sequences or two polypeptides. When positions in two sequences being compared are occupied by identical bases or amino acid monomer subunits, for example, if a position in each of two DNA molecules is occupied by adenine, the molecules are homologous at that position. The percent homology between two sequences is a function of the number of matching or homologous positions shared by the two sequences divided by the number of compared positions × 100%. For example, if 6 out of 10 positions are matched or homologous when two sequences are optimally aligned, the two sequences are 60% homologous. In general, a comparison is performed when two sequences are aligned to obtain the maximum percent homology.

"About" or "approximately" means that a numerical value is within an acceptable margin of error for the specific value determined by those of ordinary skill in the art, and the numerical value depends in part on how the value is measured or determined (i.e., the limitations of the measurement system). For example, "about" may mean a standard deviation within 1 or greater than 1. Alternatively, "about" or "substantially comprise" may mean a range of at most 20%, e.g., a variation of between 1% and 15%, between 1% and 10%, between 1% and 5%, between 0.5% and 5%, or between 0.5% and 1%. In the present disclosure, every instance where a number or numerical range is preceded by the term "about" also includes embodiments of the given number. Unless otherwise specified, when a specific value is provided in the present application and claims, the meaning of "about" or "substantially comprise" should be assumed to be within an acceptable margin of error for that specific value.

"Buffer" refers to a buffer that resists changes in pH by the action of its acid-base conjugate components. Examples of buffers that control the pH to be within an appropriate range include acetate, succinate, histidine salt, phosphate, citrate, and other organic acid buffers.

"Histidine salt buffer" is a buffer comprising histidine ions. Examples of histidine salt buffers include a histidine-hydrochloride buffer, a histidine-acetate buffer, a histidine-phosphate buffer, a histidine-sulfate buffer, etc., and the histidine-hydrochloride buffer or histidine-acetate buffer is preferred. The histidine-acetate buffer is prepared from histidine and acetic acid, and the histidine-hydrochloride buffer is prepared from histidine and histidine hydrochloride, or from histidine and hydrochloric acid.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, etc. A preferred phosphate buffer is disodium hydrogen phosphate-sodium dihydrogen phosphate.

"Pharmaceutical composition" refers to a mixture comprising one or more of the antibodies described herein and other chemical components, and the other components are, for example, physiological/pharmaceutically acceptable carriers and excipients. The purpose of the pharmaceutical composition is to maintain the stability of the active ingredient, promote administration to an organism, and facilitate the absorption of the active ingredient, thereby enabling the active ingredient to exert biological activity.

In the present disclosure, "pharmaceutical composition" and "formulation" are not mutually exclusive.

Unless otherwise specified, the solvent in a solution form of the pharmaceutical composition described in the present disclosure is water.

"Freeze-dried formulation" refers to a formulation or pharmaceutical composition obtained after lyophilizing a pharmaceutical composition in liquid or solution form or a formulation in liquid or solution form *in vacuo*.

The pharmaceutical composition described in the present disclosure can achieve a stabilizing effect: the fusion protein in the pharmaceutical composition substantially retains its physical stability and/or chemical stability and/or biological activity after storage; for example, the pharmaceutical composition substantially retains its physical and chemical stability as well as its biological activity after storage. The storage period is generally selected based on a predetermined shelf life of the pharmaceutical composition. There are currently a variety of analytical techniques for measuring protein stability, and the stability after storage for a selected period of time at a selected temperature can be measured.

A stable pharmaceutical fusion protein formulation is one in which no significant change is observed under the following conditions: storage at refrigeration temperature (2-8 °C) for at least 3 months, at least 6 months, at least 1 year, at least 2 years, or at most 3 years. In addition, stable liquid formulations include liquid formulations that exhibit desirable features after storage at 25 °C for periods including 1 month, 3 months, and 6 months, or storage at 40 °C for periods including 1 month. Typical acceptable criteria for stability are as follows: typically, no more than about 10%, preferably no more than about 5%, of fusion protein is degraded as measured by SEC-HPLC. The concentration, pH, and osmolality of the formulation have a change of no more than ±10%. Typically, clippings of no more than about 10%, preferably no more than about 5%, are observed. Typically, aggregation of no more than about 10%, preferably no more than about 5%, is formed.

A fusion protein "retains its physical stability" in a pharmaceutical formulation if it shows no significant increase in aggregation, precipitation, and/or denaturation upon visual inspection of color and/or clarity, or as determined by UV light scattering, size exclusion chromatography (SEC), and dynamic light scattering (DLS). Changes in the conformation of a protein can be evaluated by fluorescence spectroscopy (which determines the tertiary structure of the protein) and by FTIR spectroscopy (which determines the secondary structure of the protein).

A fusion protein "retains its chemical stability" in a pharmaceutical formulation if it shows no significant chemical change. Chemical stability can be assessed by detecting and quantifying proteins in chemically altered forms. Degradation processes that often alter the chemical structures of proteins include hydrolysis or clipping (evaluated by methods such as size exclusion chromatography and SDS-PAGE), oxidation (evaluated by methods such as peptide mapping in combination with mass spectroscopy or MALDI/TOF/MS), deamidation (evaluated by methods such as ion-exchange chromatography, capillary isoelectric focusing, peptide mapping, and isoaspartic acid determination), and isomerization (evaluated by isoaspartic acid content determination, peptide mapping, etc.).

A fusion protein "retains its biological activity" in a pharmaceutical formulation if the biological activity of the fusion protein at a given time is within a predetermined range of the biological activity exhibited during the preparation of the pharmaceutical formulation. The biological activity of an antibody can be determined, for example, by an antigen-binding assay.

"Administer", "give", and "treat", when applied to animals, humans, experimental subjects, cells, tissues, organs, or biological fluids, refer to contact between an exogenous drug, a therapeutic agent, a diagnostic agent, or a composition and the animals, humans, subjects, cells, tissues, organs, or biological fluids. "Administer", "give", and "treat" can refer to, for example, therapeutic, pharmacokinetic, diagnostic, research, and experimental methods. Treatment of cells comprises contact between a reagent and cells, and contact between the reagent and a fluid, wherein the fluid is in contact with the cells. "Administer", "give", and "treat" also mean treating, for example, a cell through a reagent, diagnosis, or a binding composition, or through another cell *in vitro* and *ex vivo*. "Treat", when applied to humans, veterinary, or research subjects, refers to therapeutic treatment, preventive or prophylactic measures, and research and diagnostic applications.

"Treating" or "treatment" means administering a therapeutic agent, such as a therapeutic agent comprising any one of the antibodies of the present disclosure or a pharmaceutical composition thereof, either internally or externally, to a subject who has had, is suspected of having, or is predisposed to having one or more proliferative diseases or symptoms thereof on which the therapeutic agent is known to have a therapeutic effect. Typically, the therapeutic agent is administered in an amount effective in alleviating one or more disease symptoms in the subject or population being treated, whether by inducing regression of such symptoms or inhibiting the progression of such symptoms to any clinically measurable degree. The amount of therapeutic agent effective in alleviating any specific disease symptom (also referred to as the "therapeutically effective amount") may vary depending on a variety of factors such as the disease state, age, and body weight of the subject, and the ability of the drug to produce the desired therapeutic effect in the subject. Whether a disease symptom has been palliated can be evaluated by any clinical test methods commonly used by doctors or other health care professionals to evaluate the severity or progression of the symptom. Although embodiments of the present disclosure (e.g., treatment methods or products) may be ineffective in alleviating a disease symptom of interest in a certain subject, they shall palliate the disease symptom of interest in a statistically significant number of subjects as determined by any statistical test method known in the art, such as the Student's t-test, Chi-square test, U-test by Mann and Whitney, Kruskal-Wallis test (H-test), Jonckheere-Terpstra test, and Wilcoxon test.

"Effective amount" comprises an amount sufficient to ameliorate or prevent a symptom or sign of a medical disorder. An effective amount also means an amount sufficient to allow or facilitate diagnosis. The effective amount for a subject may vary depending on factors such as the disorder to be treated, the general health of the subject, the method, route, and dose of administration, and the severity of side effects. An effective amount may be the maximum dose or administration regimen to avoid significant side effects or toxic effects. A subject of the present disclosure may be an animal or a human subject.

"Optional" or "optionally" means that the event or circumstance subsequently described may, but does not necessarily, occur. This description includes the instance where the event or circumstance occurs or does not occur.

"Subject" or "patient" means a mammal, particularly a primate, and especially a human.

Unless the context clearly requires otherwise, throughout the description and the claims, the words "comprise", "have", "include", etc., are to be understood in an inclusive sense as opposed to an exclusive or exhaustive sense, that is to say, in the sense of "including but not limited to".

**The equipment and methods used in the detection are shown below:**

### Appearance:

### A visual method was used. The sample vial was wiped clean, and the sample was observed for color, clarity, and visible foreign matter on a clarity tester against a white background and a black background at an illumination intensity of 1000-1500 lx.

Instrument for appearance examination: Jingtuo Instrument YB-2A clarity analyzer.

### SEC molecular size-exclusion chromatography:

This is an analytical method that separates a solute based on the relative relationship between the pore size of gel pores and the coil size of polymer sample molecules.

SEC monomer content percentage = A monomer/A total × 100% (A monomer represents the peak area of the main peak monomer in the sample, and A total represents the sum of the areas of all peaks).

Instrument for SEC determination: Agilent 1260 or Waters ACQUITY ARC; chromatography column: Waters, XBridge Protein BEH SEC 200Å SEC (300 × 7.8 mm, 3.5 µm)

### NR-CE capillary gel electrophoresis:

This is a type of electrophoresis in which a gel is moved into a capillary as a support medium, and a method that achieves separation under a certain voltage based on the molecular weight of the sample.

Non-reduced CE purity percentage = A main peak/A total × 100% (A main peak represents the peak area of the main peak in the sample, and A total represents the sum of the areas of all peaks).

Instrument for CE determination: Sciex model PA800 plus.

### icIEF imaged capillary isoelectric focusing electrophoresis:

This is a technique for separation according to the difference in isoelectric points (pI) of proteins. **icIEF** main peak content percentage = main peak area/total area × 100% (total area represents the sum of the areas of acidic, main, and basic peaks).

Manufacturer of instrument for **icIEF** determination: Protein Simple, model: Muarice.

### Protein concentration determination:

Instrument for protein concentration determination: ultraviolet-visible spectrophotometer; model: Nano Drop 2000; optical path length: 1 mm.

### Exemplary fusion protein pharmaceutical composition (formulation) preparation process

Step 1: A fusion protein of a human insulin analog (e.g., shown by the amino acid sequence of SEQ ID NO: 19) was mixed with the following formula amounts of auxiliary materials to prepare a formulation stock solution containing the fusion protein. After filtration, an in-process control sample was taken and tested for sterility. The stock solution was filtered through a 0.22 µm filter, and the filtrate was collected.

Step 2: The filling amount was adjusted to 1.15 mL, the filtrate was added to 2 mL vials, and the vials were stoppered. During filling, online weighing was adopted to control the difference in filling amount to be within an acceptable range.

Step 3: The capping machine was started, aluminum caps were added, and capping was carried out.

Step 4: Visual inspection was performed to confirm that the products have no defects, such as inaccurate filling amount. Vial labels were printed and attached to the vials; carton labels were printed, cartons were folded, the vials were packed into the cartons, and the carton labels were attached to the cartons.

### BRIEF DESCRIPTION OF THE DRAWINGS

FIG. 1 shows the hypoglycemic effect of a single subcutaneous injection of fusion protein 1 in a rat model of STZ-induced type I diabetes.
FIG. 2 shows the hypoglycemic effects of single subcutaneous injections of LY3209590, fusion protein 2, and fusion protein 9 in a rat model of STZ-induced type I diabetes.

### DETAILED DESCRIPTION

The present disclosure is further illustrated in detail by the following examples. These examples are for illustrative purposes only and are not intended to limit the scope of the present disclosure. Experimental methods without specific conditions indicated in the examples of the present disclosure were generally conducted under conventional conditions or conditions recommended by the manufacturer of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY. Reagents without specific sources indicated were commercially available conventional reagents.

### Examples

Preparation and purification methods for the human insulin analog-Fc fusion proteins in the present application are described in Patent Application No. WO2023174370, which can be incorporated herein by reference in its entirety.

### Example 1. Design of Human Insulin Analogs and Their Fusion Proteins with Fc

This example provides human insulin analogs of the following general formula: B₁-L₁-A₁ (from the N-terminus to the C-terminus), wherein:
B₁ is a human insulin B-chain analog with the following sequence:
FVX₁QHLCGX₂HLVEALX₃X₄VCGERGFX₅Y (SEQ ID NO: 1), wherein X₁ is selected from the group consisting of amino acid residues of Asn, Gly, and Lys, X₂ is selected from the group consisting of amino acid residues of Ser and Glu, X₃ is selected from the group consisting of amino acid residues of Tyr, His, and Glu, X₄ is selected from the group consisting of amino acid residues of His, Arg, Leu, and Glu, and X₅ is selected from the group consisting of amino acid residues of Phe and His;
A₁ is an insulin A-chain analog with the following sequence: GIVEQCCZ₁SICSLZ₂QLENYCZ₃ (SEQ ID NO: 2), wherein Z₁ is selected from the group consisting of amino acid residues of Thr, His, and Glu, Z₂ is selected from the group consisting of amino acid residues of Tyr, Asp, Ser, and Glu, and Z₃ is selected from the group consisting of amino acid residues of Gly and Asn;
L₁ is GGGGGGSGGGG (SEQ ID NO: 3) or GGGGGSGGGG (SEQ ID NO: 52).

The sequences of exemplary human insulin analogs are as follows:
> Human insulin analog 1
   FVNQHLCGEHLVEALYLVCGERGFHYGGGGGGSGGGGGIVEQCCESICSLEQLENYCG (SEQ ID NO: 4)
> Human insulin analog 2
   FVNQHLCGSHLVEALYLVCGERGFHYGGGGGGSGGGGGIVEQCCHSICSLEQLENYCG (SEQ ID NO: 5)
> Human insulin analog 3
   FVNQHLCGSHLVEALHLVCGERGFHYGGGGGGSGGGGGIVEQCCTSICSLEQLENYCN (SEQ ID NO: 6)
> Human insulin analog 4
   FVKQHLCGEHLVEALYLVCGERGFHYGGGGGGSGGGGGIVEQCCESICSLEQLENYCG (SEQ ID NO: 7)
> Human insulin analog 5
   FVNQHLCGEHLVEALYLVCGERGFHYGGGGGSGGGGGIVEQCCESICSLEQLENYCG (SEQ ID NO: 8)
> Human insulin analog 6
   FVNQHLCGEHLVEALYLVCGERGFHYGGGGGGSGGGGGIVEQCCESICSLEQLENYCN (SEQ ID NO: 9).

Further, fusion proteins of the above human insulin analogs and Fc are provided, with the fusion of the two being achieved via linkers as shown in Table 1.

**Table 1. Linker sequences for fusion of human insulin analogs with Fc**

| Amino acid sequence | Sequence No. |
|---|---|
| GGGGQGGGGQGGGGQGGGGG | SEQ ID NO:10 |
| HGGGQGGGGQGGGGQGGGGG | SEQ ID NO:11 |
| GGGGQGGGGQGGGGQGGGGQGGGGQ | SEQ ID NO:12 |
| PGPQPGPQPGPQPGPQPGPQPGPQPGPQPGPQ | SEQ ID NO:13 |
| GGGGQGGGGQGGGGQGGGGGQGGGG | SEQ ID NO:14 |
| GGGGAGGGGAGGGGAGGGGG | SEQ ID NO:15 |
| GGGGQGGGGQGGGGQGGGGQGGPCPPCPAPEAAG | SEQ ID NO:16 |

The Fc is the Fc of human IgG2, the amino acid sequences of which are as follows:
> IgG2 Fc1 
ECPPCPAPPVAGPSVFLFPPKPKDTLMISRTPEVTCVVVDVSHEDPEVQFNWYVDGVEVH
NAKTKPREEQFNSTFRVVSVLTVVHQDWLNGKEYKCKVSNKGLPAPIEKTISKTKGQPR
EPQVYTLPPSREEMTKNQVSLTCLVKGFYPSDIAVEWESNGQPENNYKTTPPMLDSDGSF
FLYSKLTVDKSRWQQGNVFSCSVMHEALHNHYTQKSLSLSPG
(SEQ ID NO: 17)
> IgG2 Fc2

GPSVFLFPPKPKDTLMISRTPEVTCVVVDVSHEDPEVQFNWYVDGVEVHNAKTKPREEQ
FNSTFRVVSVLTVVHQDWLNGKEYKCKVSNKGLPAPIEKTISKTKGQPREPQVYTLPPSR
EEMTKNQVSLTCLVKGFYPSDIAVEWESNGQPENNYKTTPPMLDSDGSFFLYSKLTVDKS
RWQQGNVFSCSVMHEALHNHYTQKSLSLSPG
(SEQ ID NO: 18).

The amino acid sequences of exemplary fusion proteins are as follows:
> Fusion protein 1 (insulin analog 1 + L2 + IgG2 Fc1) 
FVNQHLCGEHLVEALYLVCGERGFHYGGGGGSGGGGGIVEQCCESICSLEQLENYCGG
GGGQGGGQGGGQGGGGGECPPCPAPPVAGPSVFLFPPKPKDTLMISRTPEVTCVVVD
VSHEDPEVQFNWYVDGVEVHNAKTKPREEQFNSTFRVVSVLTVVHQDWLNGKEYKCK
VSNKGLPAPIEKTISKTKGQPREPQVYTLPPSREEMTKNQVSLTCLVKGFYPSDIAVEWES
NGQPENNYKTTPPMLDSDGSFFLYSKLTVDKSRWQQGNVFSCSVMHEALHNHYTQKSL
SLSPG (SEQ ID NO: 19)
> Fusion protein 2 (insulin analog 2 + L2 + IgG2 Fc1) 
FVNQHLCGSHLVEALYLVCGERGFHYGGGGGSGGGGGIVEQCCHSICSLEQLENYCGG
GGGQGGGQGGGQGGGGGECPPCPAPPVAGPSVFLFPPKPKDTLMISRTPEVTCVVVD
VSHEDPEVQFNWYVDGVEVHNAKTKPREEQFNSTFRVVSVLTVVHQDWLNGKEYKCK
VSNKGLPAPIEKTISKTKGQPREPQVYTLPPSREEMTKNQVSLTCLVKGFYPSDIAVEWES
NGQPENNYKTTPPMLDSDGSFFLYSKLTVDKSRWQQGNVFSCSVMHEALHNHYTQKSL
SLSPG (SEQ ID NO: 20)
> Fusion protein 3 (insulin analog 3 + L2 + IgG2 Fc1) 
FVNQHLCGSHLVEALHLVCGERGFHYGGGGGSGGGGGIVEQCCTSICSLEQLENYCNG
GGGQGGGQGGGGQGGGGGECPPCPAPPVAGPSVFLFPPKPKDTLMISRTPEVTCVVVD
VSHEDPEVQFNWYVDGVEVHNAKTKPREEQFNSTFRVVSVLTVVHQDWLNGKEYKCK
VSNKGLPAPIEKTISKTKGQPREPQVYTLPPSREEMTKNQVSLTCLVKGFYPSDIAVEWES
NGQPENNYKTTPPMLDSDGSFFLYSKLTVDKSRWQQGNVFSCSVMHEALHNHYTQKSL
SLSPG (SEQ ID NO: 21)
> Fusion protein 4 (insulin analog 4 + L2 + IgG2 Fc1) 
FVKQHLCGEHLVEALYLVCGERGFHYGGGGGSGGGGGIVEQCCESICSLEQLENYCGG
GGGQGGGQGGGQGGGGGECPPCPAPPVAGPSVFLFPPKPKDTLMISRTPEVTCVVVD
VSHEDPEVQFNWYVDGVEVHNAKTKPREEQFNSTFRVVSVLTVVHQDWLNGKEYKCK
VSNKGLPAPIEKTISKTKGQPREPQVYTLPPSREEMTKNQVSLTCLVKGFYPSDIAVEWES
NGQPENNYKTTPPMLDSDGSFFLYSKLTVDKSRWQQGNVFSCSVMHEALHNHYTQKSL
SLSPG (SEQ ID NO: 22)
> Fusion protein 5 (insulin analog 1 + L2 + IgG2 Fc1) 
FVNQHLCGEHLVEALYLVCGERGFHYGGGGGGSGGGGGIVEQCCESICSLEQLENYCGG
GGGQGGGGQGGGGQGGGGQGGGGQECPPCPAPPVAGPSVFLFPPKPKDTLMISRTPEVT
CVVVDVSHEDPEVQFNWYVDGVEVHNAKTKPREEQFNSTFRVVSVLTVVHQDWLNGK
EYKCKVSNKGLPAPIEKTISKTKGQPREPQVYTLPPSREEMTKNQVSLTCLVKGFYPSDIA
VEWESNGQPENNYKTTPPMLDSDGSFFLYSKLTVDKSRWQQGNVFSCSVMHEALHNHY
TQKSLSLSPG          (SEQ ID NO: 23)
> Fusion protein 6 (insulin analog 5 + L2 + IgG2 Fc1) 
FVNQHLCGEHLVEALYLVCGERGFHYGGGGSGGGGGIVEQCCESICSLEQLENYCGGG
GGQGGGGQGGGGQGGGGGECPPCPAPPVAGPSVFLFPPKPKDTLMISRTPEVTCVVVDV
SHEDPEVQFNWYVDGVEVHNAKTKPREEQFNSTFRVVSVLTVVHQDWLNGKEYKCKV
SNKGLPAPIEKTISKTKGQPREPQVYTLPPSREEMTKNQVSLTCLVKGFYPSDIAVEWESN
GQPENNYKTTPPMLDSDGSFFLYSKLTVDKSRWQQGNVFSCSVMHEALHNHYTQKSLS
LSPG (SEQ ID NO: 24)
> Fusion protein 7 (insulin analog 1 + L2 + IgG2 Fc1) 
FVNQHLCGEHLVEALYLVCGERGFHYGGGGGSGGGGGIVEQCCESICSLEQLENYCGP
GPQPGPQPGPQPGPQPGPQPGPQPGPQPGPQECPPCPAPPVAGPSVFLFPPKPKDTLMISRT
PEVTCVVVDVSHEDPEVQFNWYVDGVEVHNAKTKPREEQFNSTFRVVSVLTVVHQDW
LNGKEYKCKVSNKGLPAPIEKTISKTKGQPREPQVYTLPPSREEMTKNQVSLTCLVKGFY
PSDIAVEWESNGQPENNYKTTPPMLDSDGSFFLYSKLTVDKSRWQQGNVFSCSVMHEAL
HNHYTQKSLSLSPG (SEQ ID NO: 25)
> Fusion protein 8 (insulin analog 1 + L2 + IgG2 Fc1) 
FVNQHLCGEHLVEALYLVCGERGFHYGGGGGSGGGGGIVEQCCESICSLEQLENYCGG
GGGAGGGGAGGGGAGGGGGECPPCPAPPVAGPSVFLFPPKPKDTLMISRTPEVTCVVVD
VSHEDPEVQFNWYVDGVEVHNAKTKPREEQFNSTFRVVSVLTVVHQDWLNGKEYKCK
VSNKGLPAPIEKTISKTKGQPREPQVYTLPPSREEMTKNQVSLTCLVKGFYPSDIAVEWES
NGQPENNYKTTPPMLDSDGSFFLYSKLTVDKSRWQQGNVFSCSVMHEALHNHYTQKSL
SLSPG (SEQ ID NO: 26)
> Fusion protein 9 (insulin analog 6 + L2 + IgG2 Fc1)

FVNQHLCGEHLVEALYLVCGERGFHYGGGGGSGGGGGIVEQCCESICSLEQLENYCNH
GGGQGGGQGGGQGGGGECPPCPAPPVAGPSVFLFPPKPKDTLMISRTPEVTCVVVD
VSHEDPEVQFNWYVDGVEVHNAKTKPREEQFNSTFRVVSVLTVVHQDWLNGKEYKCK
VSNKGLPAPIEKTISKTKGQPREPQVYTLPPSREEMTKNQVSLTCLVKGFYPSDIAVEWES
NGQPENNYKTTPPMLDSDGSFFLYSKLTVDKSRWQQGNVFSCSVMHEALHNHYTQKSL
SLSPG (SEQ ID NO: 27).

In addition, LY3209590 is an ultra-long-acting insulin analog for once-weekly administration developed by Eli Lilly and Company, USA. It is currently in Phase III clinical trials, and its amino acid sequence is:

FVNQHLCGSHLVEALELVCGERGFHYGGGGGGSGGGGGIVEQCCTSTCSLDQLENYCG
GGGGQGGGGQGGGGQGGGGGECPPCPAPPVAGPSVFLFPPKPKDTLMISRTPEVTCVV
VDVSHEDPEVQFNWYVDGVEVHNAKTKPREEQFNSTFRVVSVLTVVHQDWLNGKEYK
CKVSNKGLPAPIEKTISKTKGQPREPQVYTLPPSREEMTKNQVSLTCLVKGFYPSDIAVE
WESNGQPENNYKTTPPMLDSDGSFFLYSKLTVDKSRWQQGNVFSCSVMHEALHNHYT
QKSLSLSPG (SEQ ID NO: 28)

The underlined portions are both linker sequences.

The wild-type human insulin sequences are as follows:
> A-chain
   GIVEQCCTSICSLYQLENYCN (SEQ ID NO: 29)
> B-chain
   FVNQHLCGSHLVEALYLVCGERGFFYTPKT (SEQ ID NO: 30).

### Example 2. Preparation, Characterization, and Identification of Human Insulin Analog-Fc Fusion Proteins

### 1. Preparation

### 1) Expression in HEK-293 cells

A correctly cloned plasmid was extracted using an endotoxin removal midiprep kit (Promega, A2495), and after sequencing confirmed that it was correct, preparations were made for cell transfection to produce the protein. Suspended HEK293 cells were regularly passaged using Freestyle 293 culture medium (Invitrogen, Cat. No. 12338026). One day before transfection, the cells were passaged to a density of 6 × 10⁵ cells/mL. On the day of transfection, the viability and density of cells were measured to ensure that the cell viability was >90%, and the cell density was adjusted to 1.1 × 10⁶ cells/mL. Plasmid DNA was prepared in an amount of 1 µg of DNA per mL of cells. The DNA used and the corresponding 293-fectin (Invitrogen, Cat. No. 12347019) were separately diluted using opti-MEM (Invitrogen, Cat. No. 51985-034), and the dilutions were gently shaken for thorough mixing and left to stand at room temperature for 5 min. The diluted DNA and liposome were mixed by gentle shaking, and the mixture was left to stand at room temperature for 20 min and then slowly added to the HEK293 cells to be transfected. The cell culture flask was gently rotated. The cells were then placed in a shaker at 37 °C for suspension shaking culture (5% CO₂, 135 rpm, 75% humidity). Five days after transfection, the cell culture supernatant was collected and centrifuged at 4000 rpm for 20 min, and the supernatant was collected and filtered using a 0.45 µm filter.

### 2) Expression in ExpiCHO cells

Suspended ExpiCHO cells were regularly passaged using ExpiCHO expression culture medium (Invitrogen, Cat. No. A2910002) and transfected using an ExpiCHO expression system (Cat. No. A29133), with 1 µg of DNA used per mL of transfected cells. The "high protocol" from the instructions was used. After transfection, the cells were placed in a shaker at 32 °C for suspension shaking culture (5% CO₂, 110 rpm, 75% humidity). On days 10-12 after transfection, the cell culture supernatant was collected and centrifuged at 4000 rpm for 20 min, and the supernatant was collected and filtered using a 0.45 µm filter.

### 3) Expression level determination

The OD280 of each human insulin analog-Fc fusion protein after purification was measured using an ultraviolet absorption method, and based on this, the expression levels of each fusion protein in different expression systems were calculated. The results show that fusion proteins 1 to 9 could all be expressed in the HEK293 expression system, and the yield of some of the fusion proteins was excellent. For example, the expression level of fusion protein 1 was 119 mg/L, the expression level of fusion protein 4 was 116 mg/L, the expression level of fusion protein 6 was 132 mg/L, the expression level of fusion protein 7 was 110 mg/L, and the expression level of fusion protein 8 was 119 mg/L. In the ExpiCHO transient expression system, the expression level of fusion protein 1 exceeded 400 mg/L.

### 4) Purification

### Protein-A affinity chromatography:

The cell culture supernatant expressing the Fc fusion protein was centrifuged at high speed, and the supernatant was collected and then filtered using a 0.22 µm filter membrane. A Protein-A affinity column (GE, Cat. No. 17-5474-99) was regenerated with 5 column volumes of 0.1 M aqueous NaOH solution (Sigma, Cat. No. 71687-500g) and then washed and equilibrated with 5 column volumes of 1× PBS buffer (pH 7.4) (Sangon Biotech (Shanghai) Co., Ltd., Cat. No. E607016-0500). The supernatant was loaded at a low flow rate to facilitate binding, and the flow rate was controlled to ensure a retention time of at least about 1 min. After binding, the chromatography column was rinsed with 5-10 column volumes of 1× PBS buffer (pH 7.4) until the ultraviolet absorbance fell back to baseline. Then, the sample was eluted using a 0.1 M glycine buffer (pH 3.0) (Sigma, Cat. No. 410225-250G). The elution peak was collected based on ultraviolet detection, and the eluted product was rapidly adjusted to pH 7-8 with 1 M Tris-HCl (pH 7.5) (Vetec, Cat. No. V900483) and temporarily stored. For the eluted product, solution exchange could be performed by methods well known to those skilled in the art, such as ultrafiltration concentration using an ultrafiltration tube to exchange the solution to a desired buffer system, or size exclusion (e.g., G-25 desalination) to replace the solution with a desired buffer system. The final storage system for the human insulin analog-Fc fusion protein was 1× PBS buffer (pH 7.4).

### Anion chromatography:

Firstly, the sample was diluted with an equilibration buffer of 20 mM Tris pH 8.0 (Vetec, Cat. No. V900483) to make its conductivity lower than 3 ms/cm, and a Q HP (GE, Cat. No. 17-1014-01) anion chromatography column was regenerated with 5 column volumes of 0.5 M aqueous NaOH solution (Sigma, Cat. No. 71687-500g), and then washed and equilibrated with 15 column volumes of 20 mM Tris buffer pH 8.0 (Vetec, Cat. No. V900483). The supernatant was loaded at a low flow rate to facilitate binding, and the flow rate was controlled to ensure a retention time of at least 2 min. After binding, the chromatography column was rinsed with 10 column volumes of 20 mM Tris buffer pH 8.0 (Vetec, Cat. No. V900483) until the ultraviolet absorbance fell back to baseline. 0-100% gradient elution was performed with an elution buffer of 20 mM Tris pH 8.0 (Vetec, Cat. No. V900483) and 0.5 M NaCl (Vetec, Cat. No. V900058). The elution peak was collected based on SEC-HPLC (Column: TSKgel G3000SW_{XL}, 5 µm, Cat. No. 008541) purity testing. For the eluted product, solution exchange could be performed by methods well known to those skilled in the art, such as ultrafiltration concentration using an ultrafiltration tube to exchange the solution to a desired buffer system, or size exclusion (e.g., G-25 desalination) to replace the solution with a desired buffer system. The final storage buffer system for the human insulin analog-Fc fusion protein was 1× PBS buffer (pH 7.4).

### 2. Characterization and identification

Size exclusion chromatography (SEC-HPLC): An Agilent 1260 infinity high performance liquid chromatography system and a Tosoh chromatography column (TSGel-G3000SWXL, chromatography column dimensions: 7.8 mm × 300 mm) were used. Before sample injection, the chromatography column and the system were equilibrated with 2× PBS buffer containing 5% isopropanol. Then, the sample (15 µg) was injected and eluted using the same buffer. The method duration was 20 min, and the method flow rate was 1 mL/min.

Ultra-high performance liquid chromatography-mass spectrometry: Characterization was performed using an Agilent 1290-6530 ultra-high performance liquid chromatography-mass spectrometry system and a Sepax-C4 chromatography column (particle size: 3 µm, chromatography column dimensions: 2.1 mm × 50 mm). The mobile phase A was 0.1% formic acid in water, the mobile phase B was 0.1% formic acid in acetonitrile, the flow rate was 1 mL/min, the temperature of the chromatography column was kept at 60 °C, and the chromatography gradient used is shown in Table 2. Mass spectrometer-related detection parameter settings: 1 GHz, capillary voltage: 5000 V, desolvation gas temperature: 350 °C, gas flow rate: 12 L/min, Fragmentor voltage: 380 V, MS acquisition range: 100 m/z-4000 m/z.

**Table 2. Chromatography gradient**

| | | | | | | | | | | | |
|---|---|---|---|---|---|---|---|---|---|---|---|
| RT (min) | 0 | 2 | 7 | 7.1 | 8 | 8.5 | 9 | 9.5 | 10 | 10.5 | 12 |
| B% | 10 | 10 | 50 | 90 | 90 | 10 | 90 | 10 | 90 | 10 | 10 |

The data in Table 3 show that after purification, fusion proteins 1 to 9 all had good purity and expected molecular weights.

**Table 3. SEC-HPLC purity and molecular weight**

| Fusion Protein | SEC-HPLC purity | Found molecular weight |
|---|---|---|
| LY3209590 | 100.0% | 66980.00 Da |
| Fusion protein 1 | 100.0% | 67241.36 Da |
| Fusion protein 2 | 100.0% | 67172.00 Da |
| Fusion protein 3 | 98.7% | 67164.00 Da |
| Fusion protein 4 | 99.1% | 67269.00 Da |
| Fusion protein 5 | 98.8% | 68095.00 Da |
| Fusion protein 6 | 99.3% | 67126.00 Da |
| Fusion protein 7 | 99.6% | 70602.00 Da |
| Fusion protein 8 | 99.5% | 66899.00 Da |
| Fusion protein 9 | 99.5% | 67515.00 Da |

In the following examples, the biological activity of the fusion proteins of the present disclosure was tested.

### Example 3. Assay on Type B Human Insulin Receptor (hIR-B) Autophosphorylation

The human insulin receptor subtype B autophosphorylation-promoting activity of different human insulin analog-Fc fusion proteins was investigated in CHO-K1 cells overexpressing human insulin receptor subtype B and compared. The human insulin receptor subtype B autophosphorylation-promoting activity of the fusion proteins was assessed based on their calculated EC₅₀ values in this test system. A lower EC₅₀ value indicates higher *in vitro* activity.

Experimental method: 18 h before the start of the experiment, CHO-K1-hIRB cells (Shanghai HD Biosciences, CHO-K1-hIRB-M-20170706) were plated onto a 96-well plate at 10⁴ cells/well, 100 µL of growth culture medium (F-12K culture medium, 1× penicillin-streptomycin containing 10% FBS and 4 µg/mL blasticidin) was added, and the cells were cultured overnight at 37 °C with 5% CO₂. On the morning of the day of the experiment, each human insulin analog-Fc fusion protein was diluted as required in 50 µL of starvation culture medium (F-12K culture medium, 1× penicillin-streptomycin containing 0.1% BSA and 4 µg/mL blasticidin), and after the old culture medium was discarded, the cells were incubated with the starvation culture medium containing the human insulin analog-Fc fusion protein at 37 °C with 5% CO₂ for 20 min. Then, the culture medium was discarded, and the cells were lysed using a cell lysis buffer from a kit (IR-beta phospho-Y1150/1151 kit, Cisbio, Lot No. 63ADK016PEG) and incubated at 37 °C for 30 min. Then, 16 µL of the lysate was transferred into a 96-well plate using a pipette, 4 µL of a prepared antibody mixture (containing Phospho InsuLin Receptor beta Cryptate antibody and Phospho InsuLin Receptor beta d2 antibody) was added, and the plate was incubated at room temperature for 4 h.

Data processing method: Signals were read using a Tecan microplate reader at an excitation wavelength of 320 nm and emission wavelengths of 620 nm and 665 nm. Signal ratios (665 nm/620 nm × 10,000) were calculated and fitted non-linearly to the sample concentration using a four-parameter equation in GraphPad Prism 6 to obtain EC₅₀ values. The results are shown in Table 4. In this example and subsequent examples, the control human insulin was purchased from Sigma-Aldrich.

**Table 4. The type B insulin receptor autophosphorylation activity of human insulin analog-Fc fusion proteins**

| Protein | Human insulin receptor subtype B activity (EC₅₀) |
|---|---|
| Human insulin | 0.43 nM |
| LY3209590 | 115.50 nM |
| Fusion protein 1 | 421.60 nM |
| Fusion protein 2 | 2.68 nM |
| Fusion protein 3 | 4.57 nM |
| Fusion protein 4 | 288.00 nM |
| Fusion protein 5 | 181.80 nM |
| Fusion protein 6 | 328.50 nM |
| Fusion protein 7 | 275.00 nM |
| Fusion protein 8 | 326.30 nM |
| Fusion protein 9 | 119.70 nM |

The results in Table 4 show that the fusion proteins of the present disclosure all exhibited certain levels of insulin receptor subtype B autophosphorylation-activating activity. The EC₅₀ values show that the activity of all fusion proteins was significantly weaker than that of natural human insulin, and the insulin receptor subtype B autophosphorylation-activating activity of some of the fusion proteins was comparable to or slightly stronger than that of LY3209590. This indicates that the disclosed fusion proteins are all human insulin receptor agonists, but compared to natural human insulin, the fusion proteins have reduced activity; thus, they have safer clinical administration windows, and related side effects such as hypoglycemia in patients can be reduced.

### Example 4. Assay on AKT Phosphorylation Activity in C2C12 Mouse Myoblasts

Insulin analogs, upon binding to insulin receptors, activate a series of downstream signaling pathways. The stimulation of AKT phosphorylation is a major signaling pathway through which insulin and its analogs produce hypoglycemic effects. Therefore, the AKT phosphorylation-stimulating activity of different human insulin analog-Fc fusion proteins was investigated in C2C12 mouse myoblasts and compared. The AKT phosphorylation-stimulating activity of the fusion proteins in mouse myoblasts was assessed based on their calculated EC₅₀ values in this test system. A lower EC₅₀ value indicates higher activity.

Experimental method: 18 h before the start of the experiment, C2C12 cells (Procell, CL-0044) were plated onto a 96-well plate at 10⁴ cells/well, 100 µL of growth culture medium (DMEM culture medium, 1× penicillin-streptomycin containing 10% FBS) was added, and the cells were cultured overnight at 37 °C with 5% CO₂. On the morning of the day of the experiment, each human insulin analog-Fc fusion protein was diluted as required in 50 µL of starvation culture medium (DMEM culture medium, 1× penicillin-streptomycin free of FBS), and after the old culture medium was discarded, the cells were incubated with the starvation culture medium containing the human insulin analog-Fc fusion protein in an incubator at 37 °C with 5% CO₂ for 20 min. Then, the culture medium was discarded, and the cells were lysed using a cell lysis buffer from a kit (AKT phospho-S473 kit, Cisbio, 64AKSPEG) and incubated at 37 °C for 30 min. Then, 16 µL of the lysate was transferred into a 96-well plate using a pipette, 4 µL of a prepared antibody mixture (containing Phospho-AKT Eu Cryptate antibody and Phospho-AKT d2 antibody) was added, and the plate was incubated at room temperature for 4 h. The data processing method in Example 3 was used to obtain EC₅₀ values. The results are shown in Table 5.

**Table 5. The AKT phosphorylation-stimulating activity of human insulin analog-Fc fusion proteins in C2C12 mouse myoblasts**

| Protein | AKT phosphorylation activity (EC₅₀) |
|---|---|
| Human insulin | 5.44 nM |
| LY3209590 | 193.80 nM |
| Fusion protein 1 | 489.90 nM |
| Fusion protein 2 | 90.49 nM |
| Fusion protein 3 | 28.56 nM |
| Fusion protein 4 | 565.10 nM |
| Fusion protein 5 | 320.00 nM |
| Fusion protein 6 | 455.70 nM |
| Fusion protein 7 | 280.00 nM |
| Fusion protein 8 | 543.80 nM |
| Fusion protein 9 | 132.90 nM |

The results show that the fusion proteins of the present disclosure all exhibited certain levels of insulin receptor AKT phosphorylation-activating activity. The EC₅₀ values show that the activity of all fusion proteins was significantly weaker than that of natural human insulin, and the activity of some of the fusion proteins was comparable to that of LY3209590. This indicates that the fusion proteins all exhibited significant AKT phosphorylation-stimulating activity in mouse myoblasts, but their activity was weaker than that of natural human insulin.

### Example 5. Assay on Promotion of Human MCF-7 (Human Breast Cancer Cell) Proliferation

The ability of some of the human insulin analog-Fc fusion proteins of the present disclosure to activate downstream signaling pathways to promote cell proliferation was investigated in human MCF-7 breast cancer cells. Many studies have shown that in addition to their induced hypoglycemic effects and other metabolic activities, insulin and insulin analogs also have the activity of activating other downstream signaling pathways to promote cell proliferation. The cell proliferation-promoting activity is closely related to the induction of tumorigenesis by insulin and its analogs. The later-stage development of B10 Asp insulin, a rapid-acting insulin analog developed by Novo Nordisk, was terminated because significant tumorigenesis caused by this compound was observed in a rat model. Therefore, the objective of this experiment was to use MCF-7 human breast cancer cells to assess the ability of the fusion proteins of the present disclosure to induce cell proliferation. A greatly reduced cell proliferation-promoting ability indicates a lower risk of inducing tumorigenesis in later stages. Studies have shown that the cell proliferation-promoting ability of insulin or its analogs is related to the expression of IGF-1R on the surface of some cells, and that insulin or its analogs can react with IGF-1R to promote cell proliferation, thereby inducing the possibility of tumorigenesis and tumor progression.

Experimental method: Before the start of the experiment, MCF-7 cells (Procell, CL-147) were resuspended in a DMEM culture medium containing 0.5% FBS and counted, and 100 µL of the cell suspension was added to each well of a 96-well clear-bottom black cell culture plate at a density of 5 × 10³ cells/well. The cells were cultured overnight at 37 °C with 5% CO₂. On the morning of the day of the experiment, each human insulin analog-Fc fusion protein was diluted as required in 50 µL of starvation culture medium (DMEM culture medium, containing 0.5% FBS), and after the old medium was discarded, the cells were incubated with the starvation culture medium containing the human insulin analog-Fc fusion protein at 37 °C with 5% CO₂ for 3 days. After incubation, a CellTiter-Glo assay solution (50 µL) was added to the 96-well plate, and the plate was shaken at room temperature for 2 min for thorough mixing and left to stand in a dark place for 10 min. Then, fluorescence values were read using a Tecan microplate reader, and EC₅₀ values were calculated. The results are shown in Table 6.

**Table 6. The MCF-7 proliferation-promoting activity of human insulin analog-Fc fusion proteins**

| Protein | MCF-7 proliferation-promoting activity (EC₅₀) |
|---|---|
| Human insulin | 1.32 nM |
| LY3209590 | 31.24 nM |
| Fusion protein 1 | 280.30 nM |

The results show that in multiple parallel experiments (one of which is shown in Table 6), fusion protein 1 consistently exhibited weaker MCF-7 cell proliferation-stimulating activity compared to natural human insulin. Unexpectedly, compared to LY3209590, fusion protein 1 exhibited significantly reduced MCF-7 human breast cancer cell proliferation-stimulating activity, suggesting that this fusion protein has a lower potential risk of inducing tumorigenesis. Moreover, fusion proteins 2 to 9 of the present disclosure also showed lower MCF-7 cell proliferation-promoting activity compared to human insulin. For example, the EC₅₀ value for the MCF-7 proliferation-promoting activity of fusion protein 2 was about twice the EC₅₀ value of LY3209590.

### Example 6. Assay on Pharmacokinetic Properties of Human Insulin Analog-Fc Fusion Proteins

In this example, an SD rat model was used to study the pharmacokinetic properties of a single subcutaneous or intravenous injection of fusion protein 1 in normal male SD rats.

Experimental method: Male SD rats (purchased from Vital River) weighing 170-200 g and aged 5-7 weeks were selected. Fusion protein 1 was dissolved in 1× PBS buffer, and the solution was administered to the rats via tail vein injection or dorsal subcutaneous injection at a dose of 30 nmol/kg. The rats were not fasted after administration. Blood samples were collected from the orbit at 15 min, 1 h, 2 h, and 4 h and on day 1, day 3, day 5, day 8, day 11, day 14, day 21, and day 25 after administration, anticoagulated with EDTA, and centrifuged to obtain plasma samples, which were used for the analysis of *in vivo* pharmacokinetic properties. The analysis of pharmacokinetic properties was performed using an enzyme-linked immunosorbent assay. A multi-well plate was coated with an anti-human IgG-Fc capture antibody (I2136, Sigma), and an insulin polyclonal antibody (337E2A20, Invitrogen) was added. The antibody specifically bound to the test molecule. Then, the concentrations of the test molecule in the plasma samples were determined using an anti-rabbit IgG peroxidase detection antibody (A0545, Sigma). The standard curve and samples used in the assay were prepared using 1% rat plasma (EDTA). The results are shown in Tables 7 and 8.

**Table 7. The plasma concentration-time relationship of fusion protein 1 in SD rats**

| Time | Plasma concentration (nM) | |
|---|---|---|
| | Intravenous injection | Subcutaneous injection |
| 15 min | 1455±73.0 | 2.58±0.80 |
| 1 h | 1123±11.5 | 9.71±2.12 |
| 2 h | 988±108 | 23.8±6.83 |
| 4 h | 925±42.0 | 54.7±16.0 |
| 1 day | 462±14.7 | 348±21.1 |
| 3 days | 294±11.9 | 292±10.2 |
| 5 days | 213±12.5 | 262±13.9 |
| 8 days | 136±19.0 | 190±11.1 |
| 11 days | 96.5±7.08 | 123±17.5 |
| 14 days | 81.5±14.2 | 95.1±14.5 |
| 21 days | 22.1±4.69 | 29.1±2.77 |
| 25 days | 15.6±3.29 | 17.5±1.62 |

**Table 8. Pharmacokinetic properties of fusion protein 1 in SD rats**

| Pharmacokinetic parameter | Intravenous injection | Subcutaneous injection |
|---|---|---|
| Dose (nmol/kg) | 30 | 30 |
| t1/2 (days) | 4.49 | 4.45 |
| Tₘₐₓ (days) | 0.0104 | 1 |
| Cₘₐₓ (nmol/L) | 1455 | 348 |
| AUC _{0-inf_obs} (nmol/L*d) | 3700 | 3481 |
| MRT _{0-inf_obs} (d) | 6.44 | 8.21 |
| V_{z_obs} (mL/kg) | 52.6 | 55.3 |
| Cl_{_obs} (mL/kg/d) | 8.10 | 8.62 |
| %F | NA | 94.1 |

The results show that fusion protein 1 exhibited ultra-long *in vivo* pharmacokinetic properties after administration via a single intravenous/subcutaneous injection to normal rats, and its half-life was about 4.5 days, indicating that the fusion protein may clinically have a lower frequency of administration and a longer administration interval.

### Example 7. Pharmacodynamic Study of Human Insulin Analog-Fc Fusion Proteins in Rat Model of Type I Diabetes

In this example, a rat model of streptozotocin (STZ)-induced type I diabetes was used to investigate the hypoglycemic effects and durations of action of single subcutaneous injections of some human insulin analog-Fc fusion proteins, so as to comprehensively assess their hypoglycemic activity and *in vivo* pharmacokinetic properties.

Experimental method: Male SD rats (purchased from Vital River) weighing about 350 g and aged 8 weeks were selected and acclimatized for one week. A citric acid/sodium citrate buffer with a pH range of 4.2-4.5 was prepared, and STZ was dissolved in this buffer to a concentration of 1% (w/v). The STZ solution was then injected intraperitoneally into the rats based on their actual fasting body weight at a dose of 60 mg/kg per rat. On day three after STZ injection, the fasting blood glucose levels of the rats were measured. If the found blood glucose levels were higher than 15 mM, the model establishment was considered successful.

Solutions of human insulin analog-Fc fusion proteins with corresponding concentrations were prepared using 1× PBS buffer (pH 7.4). Before administration, the blood glucose level of each rat with type I diabetes was measured, and rats with blood glucose levels between 20-30 mM were selected and randomly grouped to ensure that the average blood glucose level and the standard deviation were comparable for each group of rats (3 or 6 rats). After grouping, each group of rats was given the corresponding human insulin analog-Fc fusion protein via subcutaneous injection based on their body weight. After administration, rats were given normal access to food and water, the blood glucose levels of the animals were continuously monitored at 1 h, 2 h, 4 h, 24 h, 48 h, etc. post-dose until their blood glucose levels returned to the pre-dose blood glucose levels, and corresponding curves of blood glucose levels changing over time were plotted.

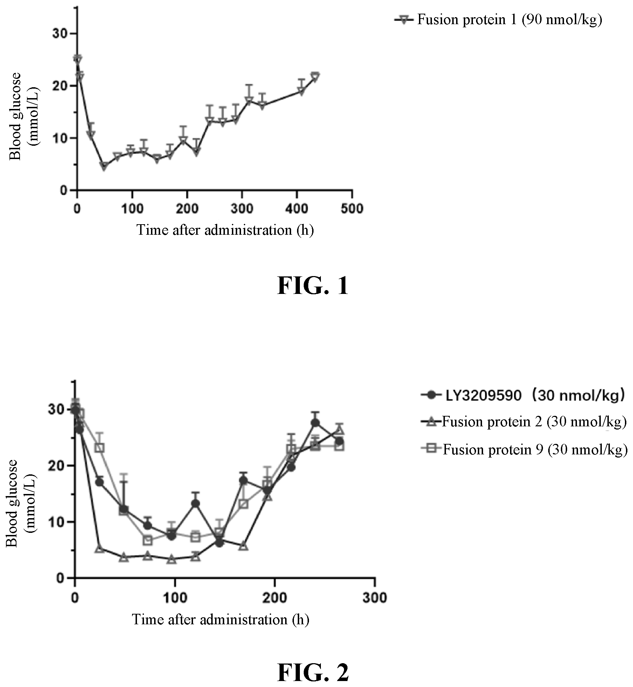

In the rat model of STZ-induced type I diabetes, the blood glucose concentration-time relationship after a single subcutaneous injection of fusion protein 1 (administered at a dose of 90 nmol/kg) is shown in FIG. 1, and the blood glucose concentration-time relationships of LY3209590, fusion protein 2, and fusion protein 9 (each administered at a dose of 30 nmol/kg) are shown in FIG. 2. The results show that fusion protein 1, fusion protein 2, and fusion protein 9 all exhibited relatively long-lasting hypoglycemic effects, and the hypoglycemic effect of the single subcutaneous injection of fusion protein 1 could last for at least 300 h.

### Example 8. Pharmacodynamic Study of Human Insulin Analog-Fc Fusion Proteins in db/db Mouse Model of Type II Diabetes

In this example, a db/db mouse model of type II diabetes was used to investigate the glycated hemoglobin-improving effects of multiple subcutaneous injections of some human insulin analog-Fc fusion proteins, so as to comprehensively assess their hypoglycemic activity and *in vivo* pharmacokinetic properties.

Experimental method: 50 db/db mice were purchased and acclimatized for 5 days, and animals with random blood glucose levels higher than 20 mmol/L were selected and grouped for an experiment. The experiment included 4 groups of 10 animals: a model control group (vehicle control group, components: 0.08 M histidine, 70 g/L sucrose, and 0.4 g/L polysorbate 80), a fusion protein 1-low dose group, a fusion protein 1-medium dose group, and a fusion protein 1-high dose group, totaling 40 animals. Dosing started on day one after grouping. Each animal was given a total of 4 doses (one dose per week) via subcutaneous injection. At the endpoint of the experiment, the glycated hemoglobin levels of all mice were measured. The method was as follows: 0.4 mL of blood was taken from the main abdominal vein of each animal, injected into an EDTA anticoagulation tube, and directly tested for glycated hemoglobin on the instrument. The results are shown in Table 9.

**Table 9. Glycated hemoglobin results in animals (%)**

| **Group** | **Administration regimen** | **RWD%** |
|---|---|---|
| Model control group | Vehicle | 11.27±0.87 |
| Fusion protein 1-low dose group | Fusion protein 1, 20 nmol/kg | 11.01±0.86 |
| Fusion protein 1-medium dose group | Fusion protein 1, 40 nmol/kg | 10.58±0.44* |
| Fusion protein 1-high dose group | Fusion protein 1, 80 nmol/kg | 10.41±0.45* |

| | | |
|---|---|---|
| Note: The results in the table are expressed as mean±SD; "*" means p ≤ 0.05 compared to the model control group. | | |

According to the experimental results, the content of glycated hemoglobin in the model control group was 11.27±0.87%, and the contents of glycated hemoglobin in the low, medium, and high dose groups of fusion protein 1 were 11.01±0.86%, 10.58±0.44% (p = 0.0481), and 10.41±0.45% (p = 0.0171), respectively; the medium dose group and the high dose group statistically differed from the model control group, and there was a certain dose-effect relationship among the three dose groups. The above results suggest that the medium and high dose groups of fusion protein 1 could significantly reduce the content of glycated hemoglobin in db/db mice, while the effect of the low dose group was not significant, and there was a certain dose-effect relationship among the three dose groups.

### Example 9. Assay on Pharmacokinetic Properties of Human Insulin Analog-Fc Fusion Proteins in Bama Pigs

In this example, a Bama pig model was used to study the pharmacokinetic properties of single subcutaneous injections of fusion protein 1 and LY3209590 in Bama pigs.

Experimental method: Male Bama pigs (Yibin Hengshu Bio-Technology Co., Ltd.) weighing 13-18 kg and aged 4-6 months were selected. Fusion protein 1 and LY3209590 were dissolved in a vehicle (components: 80 mM histidine, 70 g/L sucrose, and 0.4 g/L Tween 80) and 1× PBS buffer, respectively, and the solutions were injected subcutaneously into animals at doses of 5 nmol/kg and 2 nmol/kg, respectively. Venous blood samples (about 1 mL) were collected at 1 h, 4 h, 8 h, 24 h, 48 h, 72 h, 96 h, 120 h, 144 h, and 168 h post-dose, and were not anticoagulated. The whole blood samples were temporarily stored in an ice box and centrifuged at 2 to 8 °C at about 1800 × g for 10 min. Two tubes of serum were isolated (with about 100 µL in one tube, and the remaining serum stored in the other one) and stored at a temperature below -66 °C for the analysis of *in vivo* pharmacokinetic properties. The analysis of *in vivo* pharmacokinetic properties was performed using a method similar to that in Example 6. The specific pharmacokinetic parameters are shown in Table 10.

**Table 10. Pharmacokinetic properties of fusion protein 1 and LY3209590 in Bama pigs**

| Compound | Fusion protein 1 | LY3209590 |
|---|---|---|
| Dose (nmol/kg) | 5 | 2 |
| t1/2 (h) | 120 | 84 |
| Tₘₐₓ (h) | 19 | 25 |
| Cₘₐₓ (ng/mL) | 2900 | 1530 |
| AUC _{0-inf_obs} (h*ng/mL) | 511000 | 201000 |
| MRT_{0-inf_obs} (h) | 180 | 130 |

The results show that fusion protein 1 exhibited ultra-long *in vivo* pharmacokinetic properties after administration via a single subcutaneous injection to Bama pigs. At the doses shown in Table 10, the half-lives (t1/2) of the drugs did not change significantly with the doses. Compared to LY3209590, fusion protein 1 exhibited a significantly extended half-life, which was about 120 h, indicating that this fusion protein clinically has a lower frequency of administration and a longer administration interval.

The present disclosure provides novel fusion proteins of human insulin analogs and an IgG Fc region, which can be used to treat metabolic diseases such as type I diabetes or type II diabetes. The fusion proteins exhibited ultra-long *in vivo* pharmacokinetic properties, showed good and sustained hypoglycemic effects in the rat model of STZ-induced type I diabetes and the db/db mouse model of type II diabetes, had excellent physical/chemical stability and high expression yields, and demonstrated significantly lower *in vitro* cell proliferation-inducing activity compared to natural human insulin, suggesting that the fusion proteins have potentially good safety and a relatively low medication risk and can meet the clinical demand for ultra-long-acting insulin products.

The human insulin analog-Fc fusion protein used in the following examples was the aforementioned fusion protein-1, the amino acid sequence of which is set forth in SEQ ID NO: 19.

### Example 10. Screening for Buffer Systems and pH Values for Human Insulin Analog-Fc Fusion Protein Formulations

The following buffers were prepared, and fusion protein-1 formulations with a protein concentration of 100 mg/mL were prepared and subjected to high temperature (40 °C), repeated freeze-thaw cycle, and shaking stability studies. The experimental results are shown in Table 11.
F1: 30 mM acetic acid-histidine, pH 5.0
F2: 30 mM acetic acid-histidine, pH 5.5
F3: 30 mM acetic acid-histidine, pH 6.0
F4: 30 mM acetic acid-histidine, pH 6.5
F5: 80 mM histidine, pH 7.0

**Table 11. The results of buffer system and pH value screening**

| No. | Condition | Appearance | SEC | NR-CE | iCIEF | | |
|---|---|---|---|---|---|---|---|
| | | | Monomer% | Main peak% | Acidic peak% | Main peak% | Basic peak% |
| F1 | T0 | No particles | 98.61 | 98.49 | 22.36 | 73.21 | 4.43 |
| | Shaking-3 days | No particles | 98.54 | 98.35 | 22.84 | 73.13 | 4.04 |
| | 5 freeze-thaw cycles | No particles | 98.61 | 99.10 | 22.46 | 73.95 | 3.59 |
| | 40 °C-1 week | No particles | 97.64 | 95.73 | / | / | / |
| | 40 °C-2 weeks | No particles | 96.66 | 96.66 | / | / | / |
| | 40 °C-4 weeks | A large number of particles | 95.46 | 92.81 | / | / | / |
| F2 | T0 | No particles | 98.63 | 98.35 | 22.62 | 73.27 | 4.11 |
| | Shaking-3 days | No particles | 98.55 | 98.55 | 23.27 | 72.93 | 3.81 |
| | 5 freeze-thaw cycles | No particles | 98.62 | 98.90 | 23.14 | 73.17 | 3.69 |
| | 40 °C-1 week | A large number of particles | 97.92 | 96.23 | / | / | / |
| | 40 °C-2W | A large number of particles | / | / | / | / | / |
| | 40 °C-4W | A large number of particles | / | / | / | / | / |
| F3 | T0 | No particles | 98.70 | 99.29 | 22.81 | 72.94 | 4.25 |
| | Shaking-3 days | No particles | 98.61 | 98.62 | 23.11 | 72.89 | 4.00 |
| | 5 freeze-thaw cycles | No particles | 98.67 | 98.93 | 23.04 | 73.32 | 3.64 |
| | 40 °C-1 week | A large number of particles | 97.99 | 97.41 | / | / | / |
| | 40 °C-2 weeks | A large number of particles | / | / | / | / | / |
| | 40 °C-4 weeks | A large number of particles | / | / | / | / | / |
| F4 | T0 | No particles | 98.66 | 99.14 | 23.01 | 71.25 | 5.75 |
| | Shaking-3 days | No particles | 98.60 | 99.22 | 23.55 | 72.34 | 4.11 |
| | 5 freeze-thaw cycles | No particles | 98.64 | 99.13 | 23.08 | 73.61 | 3.32 |
| | 40 °C-1 week | A large number of particles | 98.17 | 98.14 | / | / | / |
| | 40 °C-2 weeks | A large number of particles | / | / | / | / | / |
| | 40 °C-4 weeks | A large number of particles | / | / | / | / | / |
| F5 | T0 | No particles | 98.75 | 99.15 | 23.13 | 72.62 | 4.25 |
| | Shaking-3 days | No particles | 98.67 | 98.54 | 24.28 | 71.59 | 4.14 |
| | 5 freeze-thaw cycles | No particles | 98.74 | 98.82 | 23.27 | 72.84 | 3.89 |
| | 40 °C-1 week | No particles | 98.17 | 97.49 | / | / | / |
| | 40 °C-2 weeks | No particles | 97.70 | 96.62 | / | / | / |
| | 40 °C-4 weeks | No particles | 96.97 | 95.26 | / | / | / |

### Experimental results:

The appearance results show that: after 1 week of standing at 40 °C, large numbers of visible particles appeared in formulation F2 (acetic acid-histidine buffer, pH 5.5), F3 (acetic acid-histidine buffer, pH 6.0), and F4 (acetic acid-histidine buffer, pH 6.5). After 4 weeks of standing at 40 °C, a large number of visible particles also appeared in formulation F1 (acetic acid-histidine buffer, pH 5.0), while no visible particles appeared in formulation F5 (80 mM histidine buffer, pH 7.0). The above results indicate that the histidine buffer was superior to the other buffer systems.

The SEC results show that: after 5 repeated freeze-thaw cycles and 3 days of shaking, there was no significant decrease in the SEC monomer purity of any of the formulations. After standing at a high temperature of 40 °C, the SEC monomer purity of all the different formulations decreased. After 4 weeks of standing at 40 °C, F5 (histidine buffer, pH 7.0) showed a smaller decrease (about 1.8%) than F1, indicating better stability.

The NR-CE results show that: after 5 repeated freeze-thaw cycles and 3 days of shaking, there was no significant decrease in the NR-CE main peak purity of any of the formulations. After standing at a high temperature of 40 °C, the NR-CE main peak purity of all the different formulations tended to decrease. After 4 weeks of standing at 40 °C, F5 (histidine buffer, pH 7.0) showed a smaller decrease (about 3.9%) than F1, indicating better stability.

The iCIEF results show that: after 5 repeated freeze-thaw cycles and 3 days of shaking, there was no significant decrease in the iCIEF main peak purity of any of the formulations.

In summary, the appearance, SEC, and NR-CE results collectively indicate that F5 (histidine buffer, pH 7.0) exhibited relatively good stability.

### Example 11. Screening for pH and Auxiliary Materials for Human Insulin Analog-Fc Fusion Protein Formulations

The following different formulations were prepared. Formulations containing 100 mg/mL fusion protein-1 were prepared, and their stability at a high temperature of 40 °C was investigated:
F6: 80 mM histidine pH 7.0, 8.8% (w/v) trehalose, 0.1% (w/v) poloxamer 188;
F7: 80 mM histidine + disodium hydrogen phosphate pH 7.5, 8.8% (w/v) trehalose, 0.1% (w/v) poloxamer 188;
F8: 20 mM sodium citrate pH 7.0, 8% (w/v) sucrose, 0.04% (w/v) polysorbate 80;
F9: 20 mM sodium citrate pH 7.0, 5% (w/v) mannitol, 0.04% (w/v) polysorbate 80.

**Table 12. The results of auxiliary material screening**

| No. | Condition | Appearance | SEC | NR-CE |
|---|---|---|---|---|
| | | | Monomer% | Main peak% |
| F6 | T0 | No particles | 98.77 | 98.03 |
| | 40 °C-1 week | No particles | 98.13 | 98.00 |
| | 40 °C-2 weeks | No particles | 98.16 | 98.12 |
| | 40 °C-4 weeks | No particles | 98.28 | 96.38 |
| F7 | T0 | No particles | 98.79 | 97.93 |
| | 40 °C-1 week | No particles | 98.50 | 98.13 |
| | 40 °C-2 weeks | No particles | 98.25 | 95.96 |
| | 40 °C-4 weeks | No particles | 97.58 | 93.53 |
| F8 | T0 | No particles | 98.67 | 97.90 |
| | 40 °C-1 week | A large number of particles | 98.12 | 97.56 |
| | 40 °C-2 weeks | A large number of particles | / | / |
| | 40 °C-4 weeks | A large number of particles | / | / |
| F9 | T0 | No particles | 98.65 | 97.99 |
| | 40 °C-1 week | A large number of particles | 98.04 | 97.96 |
| | 40 °C-2 weeks | A large number of particles | / | / |
| | 40 °C-4 weeks | A large number of particles | / | / |

### Experimental results

The appearance results show that: after the samples were left to stand at a high temperature of 40 °C, a large number of visible particles appeared in the formulations of the pH 7.0 citric acid system (F8 and F9) from week 1, indicating that the formulations had relatively poor stability; by contrast, no particles appeared in the formulations of the pH 7.0 and pH 7.5 histidine systems (F6 and F7) after 4 weeks of standing at a high temperature of 40 °C, indicating that the formulations were relatively stable.

The SEC results show that: after the samples were left to stand at 40 °C for 4 weeks, the stability in the case of the pH 7.0 histidine system (F6, a decrease of about 0.5% in SEC monomer purity) was slightly better than that in the case of pH 7.5 (F7, a decrease of about 1.2% in SEC monomer purity).

The NR-CE results show that: after 4 weeks of standing at 40 °C, compared to T0, there was a decrease of about 1.7% in the NR-CE main peak content of the sample in the pH 7.0 histidine system (F6), and there was a decrease of about 4.4% in the NR-CE main peak content of the sample in the pH 7.5 histidine system (F7), indicating that the stability of the sample was relatively good in the pH 7.0 histidine system (F6).

### Example 12. Screening for Bacteriostatic Agents for Fusion Protein Formulations

The following formulations containing 80 mM histidine, pH 7.0, and 100 mg/mL fusion protein were prepared and subjected to high temperature (40 °C), repeated freeze-thaw cycle, and shaking stability tests.
F10: 0.5% (w/v) benzyl alcohol, 0.02% (w/v) polysorbate 80, 4.5% (w/v) sorbitol;
F11: 1% (w/v) benzyl alcohol, 4.5% (w/v) sorbitol;
F12: 1% (w/v) benzyl alcohol, 0.02% (w/v) polysorbate 80, 4.5% (w/v) sorbitol;
F13: 1% (w/v) benzyl alcohol, 0.04% (w/v) polysorbate 80, 4.5% (w/v) sorbitol;
F14: 1% (w/v) benzyl alcohol, 0.04% (w/v) polysorbate 80, 7% (w/v) sucrose;
F15: 0.25% (w/v) phenol, 0.02% (w/v) polysorbate 80, 4.5% (w/v) sorbitol;
F16: 0.25% (w/v) phenol, 0.04% (w/v) polysorbate 80, 4.5% (w/v) sorbitol;

**Table 13. Appearances in bacteriostatic agent screening**

| **No.** | **T0** | **5 freeze-thaw cycles** | **Shaking-3 days** | **40 °C-2 weeks** | **40 °C-4 weeks** |
|---|---|---|---|---|---|
| F10 | No particles | No particles | No particles | No particles | No particles |
| F11 | No particles | No particles | No particles | A relatively large number of flocculent particles | A relatively large number of flocculent particles |
| F12 | No particles | No particles | No particles | No particles | No particles |
| F13 | No particles | No particles | No particles | No particles | No particles |
| F14 | No particles | No particles | No particles | No particles | No particles |
| F15 | No particles | No particles | No particles | No particles | A large number of particles |
| F16 | No particles | No particles | No particles | No particles | No particles |

**Table 14. SEC monomer purity in bacteriostatic agent screening**

| No. | T0 | 5 freeze-thaw cycles | Shaking-3 days | 40 °C-2 weeks | 40 °C-4 weeks |
|---|---|---|---|---|---|
| F10 | 98.42 | 98.56 | 98.45 | 97.95 | 97.33 |
| F11 | 98.46 | 98.53 | 98.48 | 97.62 | 96.63 |
| F12 | 98.46 | 98.49 | 98.44 | 97.68 | 96.58 |
| F13 | 98.45 | 98.43 | 98.48 | 97.65 | 96.76 |
| F14 | 98.36 | 98.40 | 98.11 | 97.18 | 96.09 |
| F15 | 98.45 | 98.47 | 98.4 | 97.68 | 96.95 |
| F16 | 98.46 | 98.43 | 98.36 | 97.73 | 96.80 |

**Table 15. NR-CE main peak purity in bacteriostatic agent screening**

| No. | T0 | 5 freeze-thaw cycles | Shaking-3 days | 40 °C-2 weeks | 40 °C-4 weeks |
|---|---|---|---|---|---|
| F10 | 97.99 | 98.42 | 98.24 | 96.92 | 96.45 |
| F11 | 97.99 | 98.11 | 98.13 | 96.85 | 96.22 |
| F12 | 97.99 | 98.38 | 98.36 | 96.92 | 96.08 |
| F13 | 97.92 | 98.22 | 98.17 | 96.74 | 96.10 |
| F14 | 98.02 | 98.45 | 98.09 | 96.40 | 95.86 |
| F15 | 97.99 | 98.46 | 98.31 | 96.59 | 96.21 |
| F16 | 97.94 | 98.52 | 98.25 | 96.75 | 96.08 |

**Table 16. iCIEF in bacteriostatic agent screening**

| No. | T0 | | | 5 freeze-thaw cycles | | | Shaking-3 days | | |
|---|---|---|---|---|---|---|---|---|---|
| | Acidic peak | Main peak | Basic peak | Acidic peak | Main peak | Basic peak | Acidic peak | Main peak | Basic peak |
| F10 | 20.96 | 76.96 | 2.08 | 21.69 | 76.48 | 1.83 | 21.94 | 76.18 | 1.88 |
| F11 | 21.43 | 76.2 | 2.37 | 21.6 | 76.04 | 2.35 | 22.41 | 75.37 | 2.22 |
| F12 | 21.27 | 76.5 | 2.23 | 21.46 | 76.21 | 2.33 | 22.18 | 75.34 | 2.48 |
| F13 | 21.09 | 76.99 | 1.92 | 21.78 | 75.85 | 2.37 | 22.45 | 75.69 | 1.86 |
| F14 | 22.88 | 74.99 | 2.13 | 23.67 | 74.17 | 2.16 | 24.64 | 73.21 | 2.15 |
| F15 | 21 | 76.81 | 2.19 | 21.27 | 76.93 | 1.8 | 23.03 | 75.09 | 1.88 |
| F16 | 21.18 | 76.57 | 2.25 | 21.06 | 76.81 | 2.13 | 21.92 | 76.34 | 1.74 |

### Experimental results

The appearance results show that: after 2 weeks of standing at a high temperature of 40 °C, a relatively large number of flocculent particles began to appear in formulations F11, indicating relatively poor stability; after 4 weeks of standing at 40 °C, a large number of visible particles appeared in formulation F15, indicating relatively poor stability. The other formulations all exhibited relatively good stability.

The SEC results show that: after 5 repeated freeze-thaw cycles, 3 days of shaking, and 4 weeks of standing at 40 °C, the samples all exhibited relatively good stability, their SEC monomer purity did not decrease significantly, and there was no significant difference between the formulations.

The NR-CE results show that: after 5 repeated freeze-thaw cycles, 3 days of shaking, and 4 weeks of standing at 40 °C, the samples all exhibited relatively good stability, their NR-CE main peak purity did not decrease significantly, and there was no significant difference between the formulations.

The iCIEF results show that: after the samples underwent 5 repeated freeze-thaw cycles and 3 days of shaking, the formulations all exhibited relatively good stability.

### Example 13. Other Alternative Formulations

In addition, the present disclosure further provides fusion protein pharmaceutical formulations of other formulation formulas, including but not limited to:
(1) 100 mg/mL fusion protein, 80 mg/mL sucrose, 0.4 mg/mL polysorbate 80, and 80 mM histidine buffer pH 7.0;
(2) 100 mg/mL fusion protein, 70 mg/mL sucrose, 0.2 mg/mL polysorbate 80, and 80 mM histidine buffer pH 7.0;
(3) 100 mg/mL fusion protein, 45 mg/mL sorbitol, 0.2 mg/mL polysorbate 80, and 80 mM histidine buffer pH 7.0;
(4) 100 mg/mL fusion protein, 45 mg/mL sorbitol, 0.4 mg/mL polysorbate 80, and 80 mM histidine buffer pH 7.0;
(5) 100 mg/mL fusion protein, 45 mg/mL sorbitol, 0.2 mg/mL polysorbate 80, 5 mg/mL benzyl alcohol, and 80 mM histidine buffer pH 7.0;
(6) 100 mg/mL fusion protein, 45 mg/mL sorbitol, 0.2 mg/mL polysorbate 80, 10 mg/mL benzyl alcohol, and 80 mM histidine buffer pH 7.0;
(7) 100 mg/mL fusion protein, 45 mg/mL sorbitol, 0.4 mg/mL polysorbate 80, 10 mg/mL benzyl alcohol, and 80 mM histidine buffer pH 7.0;
(8) 100 mg/mL fusion protein, 45 mg/mL sorbitol, 0.4 mg/mL polysorbate 80, 2.5 mg/mL phenol, and 80 mM histidine buffer pH 7.0;
(9) 100 mg/mL fusion protein, 70 mg/mL sucrose, 0.4 mg/mL polysorbate 80, 5 mg/mL benzyl alcohol, and 80 mM histidine buffer pH 7.0;
(10) 100 mg/mL fusion protein, 70 mg/mL sucrose, 0.4 mg/mL polysorbate 80, 10 mg/mL benzyl alcohol, and 80 mM histidine buffer pH 7.0;
(11) 100 mg/mL fusion protein, 70 mg/mL sucrose, 0.4 mg/mL polysorbate 80, 2.5 mg/mL phenol, and 80 mM histidine buffer pH 7.0; and
(12) 100 mg/mL fusion protein, 70 mg/mL sucrose, 0.4 mg/mL polysorbate 80, 5 mg/mL phenol, and 80 mM histidine buffer pH 7.0.

Experimental results show that the fusion protein formulations of the above formulation formulas all have good stability and can be applied to the preparation of fusion protein drugs.

## Claims

1. A pharmaceutical composition, comprising:
a) a fusion protein, comprising an insulin analog having the following general formula from the N-terminus to the C-terminus: B₁-L₁-A₁,
wherein:
B₁ is an insulin B-chain analog comprising the following amino acid sequence:
FVX₁QHLCGX₂HLVEALX₃X₄VCGERGFX₅Y (SEQ ID NO: 1), wherein:
X₁ is selected from the group consisting of N, G, and K,
X₂ is selected from the group consisting of S and E,
X₃ is selected from the group consisting of Y, H, and E,
X₄ is selected from the group consisting of H, R, L, and E, and
X₅ is selected from the group consisting of F and H;
A₁ is an insulin A-chain analog comprising the following amino acid sequence:
GIVEQCCZ₁SICSLZ₂QLENYCZ₃ (SEQ ID NO: 2), wherein:
Z₁ is selected from the group consisting of T, H, and E,
Z₂ is selected from the group consisting of Y, D, S, and E, and
Z₃ is selected from the group consisting of G and N;
L₁ is a linker, preferably a GS linker and/or a linker comprising at least 5 Gs, and more preferably a linker of the amino acid sequence set forth in SEQ ID NO: 3 or 52; and
b) a buffer that is one or more selected from the group consisting of histidine or a salt thereof, acetic acid or a salt thereof, succinic acid or a salt thereof, citric acid or a salt thereof, and a phosphate, preferably one or more selected from the group consisting of histidine or a salt thereof, acetic acid or a salt thereof, and a phosphate, and more preferably one or more selected from the group consisting of histidine or a salt thereof and a phosphate.

2. The pharmaceutical composition according to claim 1, wherein:
X₁ is selected from the group consisting of N and K, X₂ is selected from the group consisting of S and E, X₃ is selected from the group consisting of Y and H, X₄ is L, and X₅ is H;
Z₁ is selected from the group consisting of T, H, and E, Z₂ is E, and Z₃ is selected from the group consisting of G and N.

3. The pharmaceutical composition according to claim 1 or 2, wherein:
the insulin B-chain analog comprises or is the amino acid sequence set forth in any one of SEQ ID NOs: 31-36, and
the insulin A-chain analog comprises or is the amino acid sequence set forth in any one of SEQ ID NOs: 37-42;
preferably, the fusion protein comprises the amino acid sequence set forth in any one of SEQ ID NOs: 4-9.

4. The pharmaceutical composition according to claim 2 or 3, wherein the fusion protein further comprises C₁, and the C₁ is at least one selected from the group consisting of an Fc region of an immunoglobulin, HSA, and an HSA-binding domain;
preferably, the C₁ is an Fc region of an IgG;
more preferably, the C₁ is selected from the group consisting of Fc regions of human IgG1, IgG2, and IgG4.

5. The pharmaceutical composition according to claim 4, wherein the C₁ is located at the N-terminus or C-terminus of the insulin analog, preferably at the C-terminus of the insulin analog.

6. The pharmaceutical composition according to claim 4 or 5, wherein the C₁ comprises or is the amino acid sequence set forth in any one of SEQ ID NOs: 17, 18, and 48-51, or an amino acid sequence having at least 90% identity thereto.

7. The pharmaceutical composition according to any one of claims 4 to 6, wherein the fusion protein further comprises a linker L₂, and C₁ forms the fusion protein with B₁-L₁-A₁ via L₂;
preferably, the L₂ is selected from the group consisting of (GₘQᵢ)ₙ, (GₘAᵢ)ₙ, (GₘQ)ₙ, (GₘA)ₙ, and (PGPQ)ₛ, wherein:
each m is independently selected from the group consisting of integers from 1 to 10,
each n is independently selected from the group consisting of integers from 1 to 10, and
each i is independently selected from the group consisting of 0-4;
s is selected from the group consisting of integers from 1 to 10;
more preferably, the L₂ comprises or is the amino acid sequence set forth in any one of SEQ ID NOs: 10-16.

8. The pharmaceutical composition according to any one of claims 1 to 7, wherein the fusion protein comprises or is the amino acid sequence set forth in any one of SEQ ID NOs: 19-27, or an amino acid sequence having at least 90% sequence identity thereto.

9. The pharmaceutical composition according to any one of claims 1 to 8, wherein the fusion protein is a dimer, preferably a homodimer.

10. The pharmaceutical composition according to any one of claims 1 to 9, wherein the insulin analog is an insulin receptor agonist with insulin receptor agonistic activity.

11. The pharmaceutical composition according to any one of claims 1 to 10, further comprising a surfactant, wherein preferably, the surfactant is one or more selected from the group consisting of polysorbate and poloxamer; more preferably, the surfactant is polysorbate 80 or poloxamer 188.

12. The pharmaceutical composition according to any one of claims 1 to 11, further comprising one or more of a saccharide and a polyol, wherein preferably, the saccharide is one or more selected from the group consisting of sucrose and trehalose, and the polyol is one or more selected from the group consisting of mannitol, sorbitol, and glycerol; more preferably, the polyol is sorbitol or glycerol, and the saccharide is sucrose.

13. The pharmaceutical composition according to any one of claims 1 to 12, further comprising a bacteriostatic agent, wherein preferably, the bacteriostatic agent comprises one or more of benzyl alcohol, phenol, and m-cresol.

14. The pharmaceutical composition according to any one of claims 1 to 13, wherein the pharmaceutical composition has a pH of 5-9.5, preferably 6-9, more preferably 6.5-8.5, and most preferably 6.5-7.5.

15. The pharmaceutical composition according to any one of claims 1 to 14, wherein the fusion protein is at a concentration of 0.01 mg/mL-500 mg/mL, preferably 0.1 mg/mL-400 mg/mL, more preferably 0.5 mg/mL-200 mg/mL, and most preferably 1 mg/mL-150 mg/mL.

16. The pharmaceutical composition according to any one of claims 1 to 15, wherein the buffer is at a concentration of 1 mM-200 mM, preferably 5 mM-150 mM, more preferably 10 mM-120 mM, and most preferably 10 mM-100 mM.

17. The pharmaceutical composition according to any one of claims 11 to 16, wherein the surfactant is at a concentration of 0.01 mg/mL-5 mg/mL, preferably 0.05 mg/mL-3 mg/mL, more preferably 0.08 mg/mL-1.5 mg/mL, and most preferably 0.1 mg/mL-1 mg/mL.

18. The pharmaceutical composition according to any one of claims 12 to 17, wherein the polyol or saccharide is at a concentration of 1 mg/mL-200 mg/mL, preferably 10 mg/mL-150 mg/mL. more preferably 30 mg/mL-120 mg/mL, and most preferably 40 mg/mL-100 mg/mL.

19. The pharmaceutical composition according to any one of claims 13 to 18, wherein the bacteriostatic agent is at a concentration of 0.01 mg/mL-50 mg/mL, preferably 0.05 mg/mL-40 mg/mL. more preferably 0.1 mg/mL-30 mg/mL, and most preferably 0.5 mg/mL-20 mg/mL.

20. A pharmaceutical composition, comprising:
the fusion protein according to any one of claims 1 to 10,
a buffer that is one or more selected from the group consisting of histidine or a salt thereof and a phosphate, preferably one or more selected from the group consisting of histidine and disodium hydrogen phosphate,
polysorbate or poloxamer, and
sucrose, sorbitol, or glycerol,
wherein optionally, the composition further comprises a bacteriostatic agent; preferably, the bacteriostatic agent comprises one or more of benzyl alcohol, phenol, and m-cresol.

21. The pharmaceutical composition according to claim 20, comprising any one of the following groups 1)-4):
1) 0.01 mg/mL-500 mg/mL said fusion protein according to any one of claims 1 to 10,
1 mM-200 mM buffer that is one or more selected from the group consisting of histidine or a salt thereof and a phosphate, preferably one or more selected from the group consisting of histidine and disodium hydrogen phosphate,
0.01 mg/mL-5 mg/mL polysorbate or poloxamer, and
1 mg/mL-200 mg/mL sucrose or sorbitol;
optionally, the pharmaceutical composition further comprising 0.01 mg/mL-50 mg/mL bacteriostatic agent comprising one or more of benzyl alcohol, phenol, and m-cresol;
and the pharmaceutical composition having a pH of 5-9.5;
2) 0.1 mg/mL-400 mg/mL said fusion protein according to any one of claims 1 to 10,
5 mM-150 mM buffer that is one or more selected from the group consisting of histidine or a salt thereof and a phosphate, preferably one or more selected from the group consisting of histidine and disodium hydrogen phosphate,
0.05 mg/mL-3 mg/mL polysorbate or poloxamer, and
10 mg/mL-150 mg/mL sucrose or sorbitol;
optionally, the pharmaceutical composition further comprising 0.05 mg/mL-40 mg/mL bacteriostatic agent comprising one or more of benzyl alcohol, phenol, and m-cresol;
and the pharmaceutical composition having a pH of 6-9;
3) 0.5 mg/mL-200 mg/mL said fusion protein according to any one of claims 1 to 10,
10 mM-120 mM buffer that is one or more selected from the group consisting of histidine or a salt thereof and a phosphate, preferably one or more selected from the group consisting of histidine and disodium hydrogen phosphate,
0.08 mg/mL-1.5 mg/mL polysorbate or poloxamer, and
30 mg/mL-120 mg/mL sucrose or sorbitol;
optionally, the pharmaceutical composition further comprising 0.1 mg/mL-30 mg/mL bacteriostatic agent comprising one or more of benzyl alcohol, phenol, and m-cresol;
and the pharmaceutical composition having a pH of 6.5-8.5; and
4) 1 mg/mL-150 mg/mL said fusion protein according to any one of claims 1 to 10,
10 mM-100 mM buffer that is one or more selected from the group consisting of histidine or a salt thereof and a phosphate, preferably one or more selected from the group consisting of histidine and disodium hydrogen phosphate,
0.1 mg/mL-1 mg/mL polysorbate or poloxamer, and
40 mg/mL-100 mg/mL sucrose or sorbitol;
optionally, the pharmaceutical composition further comprising 0.5 mg/mL-20 mg/mL bacteriostatic agent comprising one or more of benzyl alcohol, phenol, and m-cresol;
and the pharmaceutical composition having a pH of 6.5-7.5.

22. The pharmaceutical composition according to claim 21, comprising any one of the following groups 1)-13):
1) 1-200 mg/mL said fusion protein according to any one of claims 1 to 10,
about 80 mM or about 40 mM histidine,
0.1 mg/mL-1 mg/mL polysorbate 80 or poloxamer 188, and
about 70 mg/mL sucrose, about 60 mg/mL sucrose, or about 45 mg/mL sorbitol;
the pharmaceutical composition having a pH of 6.5-7.5; and
optionally, the pharmaceutical composition further comprising 0.5 mg/mL-20 mg/mL bacteriostatic agent comprising one or more of benzyl alcohol, phenol, and m-cresol;
2) 1-200 mg/mL said fusion protein according to any one of claims 1 to 10, preferably about 1 mg/mL, about 20 mg/mL, about 40 mg/mL, about 50 mg/mL, about 60 mg/mL, about 80 mg/mL, about 100 mg/mL, about 120 mg/mL, or 150 mg/mL,
about 80 mM histidine,
about 0.2 mg/mL or about 0.4 mg/mL polysorbate 80, and
about 70 mg/mL sucrose or about 45 mg/mL sorbitol;
the pharmaceutical composition having a pH of about 6.5, about 7.0, or about 7.5; and
optionally, the pharmaceutical composition further comprising a bacteriostatic agent comprising about 5 mg/mL or about 10 mg/mL benzyl alcohol, about 2.5 mg/mL or about 5 mg/mL phenol, or about 1 mg/mL or about 3 mg/mL m-cresol;
3) about 100 mg/mL said fusion protein according to any one of claims 1 to 10,
about 80 mM histidine,
about 0.2 mg/mL polysorbate 80, and
about 70 mg/mL sucrose;
the pharmaceutical composition having a pH of about 7.0; and
optionally, the pharmaceutical composition further comprising a bacteriostatic agent comprising about 5 mg/mL or about 10 mg/mL benzyl alcohol, about 2.5 mg/mL or about 5 mg/mL phenol, or about 1 mg/mL or about 3 mg/mL m-cresol;
4) about 100 mg/mL said fusion protein according to any one of claims 1 to 10,
about 80 mM histidine,
about 0.4 mg/mL polysorbate 80, and
about 70 mg/mL sucrose;
the pharmaceutical composition having a pH of about 7.0; and
optionally, the pharmaceutical composition further comprising a bacteriostatic agent comprising about 5 mg/mL or about 10 mg/mL benzyl alcohol, about 2.5 mg/mL or about 5 mg/mL phenol, or about 1 mg/mL or about 3 mg/mL m-cresol;
5) about 100 mg/mL said fusion protein according to any one of claims 1 to 10,
about 80 mM histidine,
about 0.2 mg/mL polysorbate 80, and
about 45 mg/mL sorbitol;
the pharmaceutical composition having a pH of about 7.0; and
optionally, the pharmaceutical composition further comprising a bacteriostatic agent comprising about 5 mg/mL or about 10 mg/mL benzyl alcohol, about 2.5 mg/mL or about 5 mg/mL phenol, or about 1 mg/mL or about 3 mg/mL m-cresol;
6) about 100 mg/mL said fusion protein according to any one of claims 1 to 10,
about 80 mM histidine,
about 0.4 mg/mL polysorbate 80, and
about 45 mg/mL sorbitol;
the pharmaceutical composition having a pH of about 7.0; and
optionally, the pharmaceutical composition further comprising a bacteriostatic agent comprising about 5 mg/mL or about 10 mg/mL benzyl alcohol, about 2.5 mg/mL or about 5 mg/mL phenol, or about 1 mg/mL or about 3 mg/mL m-cresol;
7) about 100 mg/mL said fusion protein according to any one of claims 1 to 10,
about 80 mM histidine,
about 0.2 mg/mL polysorbate 80, and
about 70 mg/mL sucrose;
the pharmaceutical composition having a pH of about 7.5; and
optionally, the pharmaceutical composition further comprising a bacteriostatic agent comprising about 5 mg/mL or about 10 mg/mL benzyl alcohol, about 2.5 mg/mL or about 5 mg/mL phenol, or about 1 mg/mL or about 3 mg/mL m-cresol;
8) about 100 mg/mL said fusion protein according to any one of claims 1 to 10,
about 80 mM histidine,
about 0.4 mg/mL polysorbate 80, and
about 70 mg/mL sucrose;
the pharmaceutical composition having a pH of about 7.5; and
optionally, the pharmaceutical composition further comprising a bacteriostatic agent comprising about 5 mg/mL or about 10 mg/mL benzyl alcohol, about 2.5 mg/mL or about 5 mg/mL phenol, or about 1 mg/mL or about 3 mg/mL m-cresol;
9) about 100 mg/mL said fusion protein according to any one of claims 1 to 10,
about 80 mM histidine,
about 0.2 mg/mL polysorbate 80, and
about 45 mg/mL sorbitol;
the pharmaceutical composition having a pH of about 7.5; and
optionally, the pharmaceutical composition further comprising a bacteriostatic agent comprising about 5 mg/mL or about 10 mg/mL benzyl alcohol, about 2.5 mg/mL or about 5 mg/mL phenol, or about 1 mg/mL or about 3 mg/mL m-cresol;
10) about 100 mg/mL said fusion protein according to any one of claims 1 to 10,
about 80 mM histidine,
about 0.4 mg/mL polysorbate 80, and
about 45 mg/mL sorbitol;
the pharmaceutical composition having a pH of about 7.5; and
optionally, the pharmaceutical composition further comprising a bacteriostatic agent comprising about 5 mg/mL or about 10 mg/mL benzyl alcohol, about 2.5 mg/mL or about 5 mg/mL phenol, or about 1 mg/mL or about 3 mg/mL m-cresol;
11) 1-200 mg/mL said fusion protein according to any one of claims 1 to 10, preferably about 1 mg/mL, about 20 mg/mL, about 40 mg/mL, about 50 mg/mL, about 60 mg/mL, about 80 mg/mL, about 100 mg/mL, about 120 mg/mL, or 150 mg/mL,
about 40 mM histidine,
about 0.4 mg/mL poloxamer 188, and
about 60 mg/mL sucrose;
the pharmaceutical composition having a pH of about 6.5, about 7.0, or about 7.5; and
optionally, the pharmaceutical composition further comprising one or more of benzyl alcohol, phenol, and m-cresol, preferably comprising about 5 mg/mL or about 10 mg/mL benzyl alcohol, about 2.5 mg/mL or about 5 mg/mL phenol, or about 1 mg/mL or about 3 mg/mL m-cresol;
12) about 40 mg/mL or about 60 mg/mL said fusion protein according to any one of claims 1-10, about 40 mM histidine,
about 0.4 mg/mL poloxamer 188, and
about 60 mg/mL sucrose;
the pharmaceutical composition having a pH of about 7.0; and
optionally, the pharmaceutical composition further comprising one or more of benzyl alcohol, phenol, and m-cresol, preferably comprising about 5 mg/mL or about 10 mg/mL benzyl alcohol, about 2.5 mg/mL or about 5 mg/mL phenol, or about 1 mg/mL or about 3 mg/mL m-cresol; and
13) about 40 mg/mL or about 60 mg/mL said fusion protein according to any one of claims 1-10, about 40 mM histidine,
about 0.4 mg/mL poloxamer 188, and
about 60 mg/mL sucrose;
the pharmaceutical composition having a pH of about 7.5; and
optionally, the pharmaceutical composition further comprising one or more of benzyl alcohol, phenol, and m-cresol, preferably comprising about 5 mg/mL or about 10 mg/mL benzyl alcohol, about 2.5 mg/mL or about 5 mg/mL phenol, or about 1 mg/mL or about 3 mg/mL m-cresol.

23. A freeze-dried formulation, wherein the freeze-dried formulation is capable of forming the pharmaceutical composition according to any one of claims 1 to 22 after reconstitution, or the freeze-dried formulation is obtained by lyophilizing the pharmaceutical composition according to any one of claims 1 to 22.

24. A reconstituted solution, wherein the reconstituted solution is prepared by reconstituting the freeze-dried formulation according to claim 23.

25. A product, comprising a container, wherein the container contains the pharmaceutical composition according to any one of claims 1 to 22, the freeze-dried formulation according to claim 23, or the reconstituted solution according to claim 24.

26. Use of the pharmaceutical composition according to any one of claims 1 to 22, the freeze-dried formulation according to claim 23, or the reconstituted solution according to claim 24 in the manufacture of a medicament for treating diabetes and a complication thereof or lowering a blood glucose level, wherein:
preferably, the diabetes and the complication thereof are selected from the group consisting of:
type I diabetes, type II diabetes, and complications thereof;
more preferably, the complication is selected from the group consisting of diabetes-related heart disease, stroke, retinopathy, neuropathy, and kidney disease
